# EUROPÄISCHE PATENTANMELDUNG

(11) **EP 3 536 794 A2**
(43) Veröffentlichungstag der Anmeldung: **11.09.2019**
(21) Anmeldenummer: 19153543.4
(22) Anmeldetag: 06.08.2014
(51) Int. Cl.: C12N 15/86, C12N 15/10, A61K 48/00

(54) **PEPTIDE MIT SPEZIFITÄT FÜR DIE LUNGE**

(30) Priorität: 09.08.2013 DE 102013215817
(62) Teilanmeldung aus: 14753039.8
(71) Anmelder: Boehringer Ingelheim International GmbH, 55216 Ingelheim am Rhein (DE)
(72) Erfinder: TREPEL, Martin, 22149 Hamburg (DE); KOERBELIN, Jakob, 22769 Hamburg (DE); MICHELFELDER, Stefan, 79211 Denzlingen (DE)
(74) Vertreter: Uexküll & Stolberg

(57) **Zusammenfassung**

Die Erfindung betrifft ein Peptid, Polypeptid oder Protein, das spezifisch an Zellen des Lungenendothels bindet. Das Peptid, Polypeptid oder Protein kann Bestandteil eines viralen Kapsids sein und dazu verwendet werden, einen rekombinanten viralen Vektor nach systemischer Verabreichung an ein Subjekt selektiv zum Lungenendothelgewebe zu leiten und dort für eine gewebespezifische Expression von einem oder mehreren Transgenen zu sorgen. Die Erfindung betrifft somit auch einen rekombinanten viralen Vektor, vorzugsweise einen AAV-Vektor, der ein Kapsid umfasst, welches das erfindungsgemäße Peptid, Polypeptid oder Protein enthält, sowie mindestens ein in dem Kapsid verpacktes Transgen. Der virale Vektor eignet sich insbesondere zur therapeutischen Behandlung einer Lungenfunktionsstörung oder einer Lungenerkrankung. Die Erfindung betrifft ferner Zellen und pharmazeutische Zusammensetzungen, die den erfindungsgemäßen viralen Vektor umfassen.

## Beschreibung

Die Erfindung betrifft ein Peptid, Polypeptid oder Protein, das spezifisch an Zellen des Lungenendothels bindet. Das Peptid, Polypeptid oder Protein kann Bestandteil eines viralen Kapsids sein und dazu verwendet werden, einen rekombinanten viralen Vektor nach systemischer Verabreichung an ein Subjekt selektiv zum Lungenendothelgewebe zu leiten und dort für eine gewebespezifische Expression von einem oder mehreren Transgenen zu sorgen. Die Erfindung betrifft somit auch einen rekombinanten viralen Vektor, vorzugsweise einen AAV-Vektor, der ein Kapsid umfasst, welches das erfindungsgemäße Peptid, Polypeptid oder Protein enthält, sowie mindestens ein in dem Kapsid verpacktes Transgen. Der virale Vektor eignet sich insbesondere zur therapeutischen Behandlung einer Lungenfunktionsstörung oder einer Lungenerkrankung. Die Erfindung betrifft ferner Zellen und pharmazeutische Zusammensetzungen, die den erfindungsgemäßen viralen Vektor umfassen.

### HINTERGRUND DER ERFINDUNG

Die pulmonale Hypertonie ist eine schwere chronische Erkrankung der Lunge, die unbehandelt regelmäßig zum Tode führt. Der Begriff fasst Krankheiten verschiedener Ursachen zusammen, die durch eine strukturelle Veränderung der pulmonalen Vaskulatur charakterisiert sind und bei denen es zu einem Anstieg des Blutdrucks im pulmonal-arteriellen System auf mehr als 25 mm Hg kommt [1]. Dies führt bei betroffenen Patienten in der Regel zu einer belastungsabhängigen Atemnot und einer generellen Leistungsminderung. Mit fortschreitender Erkrankung kommt es zu einer Verengung der Gefäße, die durch eine Umwandlung (Remodeling) und Verdickung aller drei Schichten der Gefäßwände, d.h. Intima, Media und Adventitia, verursacht wird [2]. Damit kommt es oft zu Ruhe-Dyspnoe, globaler respiratorischer Insuffizienz und den mit einer Rechtsherzinsuffizienz einhergehenden Stauungssyndromen sowie langfristig zum Herzversagen. Die pulmonal-arteriellen Hypertonie ist eine besonders schwere Form der pulmonalen Hypertonie, bei der das mittlere Überleben ab Diagnose, die auf Grund der anfänglich schwachen Symptome oft sehr spät gestellt wird, bei lediglich etwa drei Jahren liegt [3].

Zur Erforschung der Mechanismen der pulmonalen Hypertonie und für präklinische therapeutische Untersuchungen stehen verschiedene Tiermodelle zur Verfügung, die unterschiedliche Charakteristika der Erkrankung abbilden. Dabei kann zwischen induzierbaren Modellen (z.B. durch Hypoxie, Monocrotalin oder Antigene) und transgenen Modellen unterschieden werden, wobei die Wahl des geeigneten Modells von der zu untersuchenden Fragestellung abhängt [4].

Nicht alle möglichen Ursachen der verschiedenen Formen der pulmonal-arteriellen Hypertonie sind bis heute aufgeklärt. Trotzdem gibt es mehrere bekannte, auch therapeutisch relevante Faktoren. So wird z.B. bei der idiopathischen pulmonal-arteriellen Hypertonie die vermehrte Freisetzung vasokonstriktiver Faktoren diskutiert [5-7], während in vielen Fällen der familiären pulmonal-arteriellen Hypertonie Mutationen des BMPR2 [8] oder des Activin-Receptor-Like-Kinase-1 (ALK1)-Gens [9] als ursächlich erachtet werden.

Die Entwicklung neuer Therapieoptionen für die Behandlung der pulmonalen Hypertonie bzw. der pulmonal-arteriellen Hypertonie ist dringend erforderlich. Eine solche könnte der Transfer therapeutischer Gene in Lungengewebe, und insbesondere in das Lungenendothel, sein. Vektoren, die einen spezifischen und effizienten Gentransfer in das Lungenendothel ermöglichen, sind jedoch im Stand der Technik bislang nicht beschrieben worden. Die gentherapeutische Behandlung mittels viraler Vektoren stellt eine vielversprechende Behandlungsmöglichkeit für Erkrankungen dar, die auf eine konventionelle Behandlung nicht oder nicht in ausreichendem Maße ansprechen. Dieser Ansatz beruht auf der Einschleusung von therapeutischen Genen in den zu behandelnden Organismus mit Hilfe von Viren, die so modifizierte wurden, dass sie die Sequenz des entsprechenden Gens in ihrem Genom aufweisen. Virale Vektoren, die im Rahmen der Gentherapie bereits für gentherapeutische Therapieansätze verwendet wurden, basieren auf Retroviren, Lentiviren, Adenoviren und Adeno-assoziierte Viren.

Adeno-assozierte Viren (AAV) stellen vielversprechende Kandidaten für den Einsatz in der der klinischen Praxis dar, da sie als verhältnismäßig sicher eingestuft werden. AAV-Vektoren sind in der Lage, ein Transgen in ein Gewebe einzuschleusen und dort stabil und effizient zu exprimieren. Gleichzeitig weisen diese Vektoren keine bekannten Pathogenitätsmechanismen auf [10]. Besondere Bedeutung für eine klinische Anwendung kommt den AAV-Vektoren vom Serotyp 2 (AAV2) zu, die als besonders gut untersucht gelten. Nach Einschleusung durch AAV-Vektoren können die Transgene in verschiedenen Formen in der transfizierten Zelle vorliegen, z.B. als episomale, einzel- oder doppelsträngige DNA. Auch konkatamere Formen der DNA wurden in transduzierten Zellen nachgewiesen.

Das Genom von AAV2 wird von einem linearen, einzelsträngigen DNA-Molekül von etwa 4700 Nukleotiden Länge gebildet und umfasst an beiden Enden Inverted Terminal Repeats (ITRs). Das Genom umfasst ferner zwei große offene Leserahmen, die als Replikations-Region (rep) und Kapsid-Region (cap) bezeichnet werden. Die Replikations-Region kodiert für Proteine, die im Rahmen der Virusreplikation erforderlich sind. Die Kapsid-Region hingegen kodiert für die strukturellen Proteine VP1, VP2 und VP3, aus denen sich das ikosaedrische Kapsid des Virus zusammensetzt.

Wie die meisten im Stand der Technik bekannten, gentherapeutisch verwendbaren Vektoren allerdings weisen Wildtyp-AAV-Vektoren, wie z.B. die beschriebenen AAV2-Vektoren, keine ausreichende Spezifität für ein bestimmtes Gewebe auf, sondern infizieren eine große Bandbreite von Zelltypen. Bei systemischer Gabe dieser Wildtyp-Vektoren kommt es somit zu einer unzureichenden Transduktion von Lungengewebe, während aufgrund der unerwünschten Transduktion von anderen Geweben mit starken Immunreaktionen im behandelten Patienten gerechnet werden muss. Fortschritte bei der Entwicklung von viralen Vektoren, die eine erhöhte Spezifität für bestimmte Organe aufweisen, wurden in der Vergangenheit durch den Einsatz von Peptidliganden erreicht, die in der Lage sind, die Vektoren zu einem bestimmten Organ zu leiten [11-12]. Es konnte gezeigt werden, dass bestimmte Peptidliganden für ein "Homing" zu verschiedenen Organen wie z.B. zum Gehirn sorgen.

Literaturstelle [13] beschreibt ein Verfahren, welches das Screening nach Kapsiden von AAV2 mit modifiziertem Tropismus in randomisierten Peptidbibliotheken erlaubt. Aus diesen Bibliotheken können Vektoren isoliert werden, die *in vitro* spezifisch einen gewünschten Zelltyp transduzieren. Jedoch wurde überraschenderweise festgestellt, dass die auf diese Weise selektierten Kapside oftmals für eine Verwendung *in vivo* ungeeignet sind, da ihnen im Tiermodell die nötige Spezifität fehlte [14].

Es besteht weiterhin ein großes Bedürfnis nach Mitteln, die in der Lage sind, den Tropismus viraler Vektoren zu modulieren und damit für eine ausreichende Zell- oder Gewebespezifität sorgen, die einen gezielten Transport eines viralen Vektors in die Lunge ermöglicht. Solche Vektoren können für eine spezifische Expression von therapeutischen Genen in Lungengewebe sorgen, um auf diese Weise Erkrankungen und/oder Funktionsstörungen der Lunge wirksam zu behandeln.

Die vorliegende Erfindung stellt virale Vektoren für den zielgerichteten Gentransfer in die Lunge bereit. Die erfindungsgemäßen viralen Vektoren exprimieren auf ihrer Kapsid-Oberfläche eine bislang unbekannte Aminosäuresequenz, die *in vivo* spezifisch von Rezeptoren auf dem Endothelgewebe der Lunge erkannt wird. Somit transduzieren die viralen Vektoren der vorliegenden Erfindung nach systemischer Verabreichung an ein Subjekt spezifisch dessen Lungengewebe.

Die erfindungsgemäßen viralen Vektoren ermöglichen darüber hinaus eine starke und lang anhaltende Expression eines Transgens in den endothelialen Zellen der Lunge bei nur geringfügiger Immunreaktion und sind daher insbesondere für die gentherapeutische Behandlung von bestimmten Lungenfunktionsstörungen und/oder Lungenerkrankungen geeignet. Nach der Transfektion rufen die AAV-Vektoren lediglich eine geringfügige Immunreaktion in dem Wirt hervor und sind somit besonders für die Gentherapie geeignet.

### BESCHREIBUNG DER ERFINDUNG

Im Rahmen der vorliegenden Erfindung wurden durch Selektion in einer randomisierten AAV2-Heptamer-Pepidbibliothek verschiedene lungenspezifische Sequenzen identifiziert. Auf Basis der identifizierten Peptidsequenz ESGHGYF (SEQ ID NO:2) wurde der rekombinante virale Vektor rAAV2-ESGHGYF hergestellt, bei dem die Peptidsequenz ESGHGYF als Teilsequenz des Kapsid-Proteins VP1 exprimiert wird. Anschließend wurde der Vektor rAAV2-ESGHGYF intravenös an Mäuse verabreicht, wobei sowohl *in vitro* als auch *in vivo* eine eindeutige Spezifität des Vektors für Lungenendothelgewebe beobachtet werden konnte. Die Spezifität konnte durch Färbung mit CD31 nachgewiesen werden, wie es in den vorliegenden Beispielen beschrieben wird. Darüber hinaus konnte durch Austausch der Aminosäuren Glutaminsäure (E) und Serin (S) am N-Terminus des Peptides in einem Alanin-Scan festgestellt werden, dass diese beiden Aminosäuren für die Spezifität der Transduktion nicht relevant sind, sodass lediglich die Kernstruktur GHGYF (SEQ ID NO:1) für die Lungenspezifität verantwortlich ist.

Die vorliegende Erfindung stellt daher verschiedene lungenspezifische Peptidsequenzen bereit, die in besonderer Weise dazu geeignet sind, therapeutische Mittel, wie z.B. virale Vektoren, gezielt in die Lunge eines zu behandelnden Subjekts zu leiten.

In einem ersten Aspekt betrifft die vorliegende Erfindung demgemäß ein Peptid, Polypeptid oder Protein, das spezifisch an Endothelzellen der Lunge bindet, wobei das Peptid, Polypeptid oder Protein die Aminosäuresequenz von SEQ ID NO:1 umfasst. Vorzugsweise umfasst das Peptid, Polypeptid oder Protein die Aminosäuresequenz von SEQ ID NO:2 oder eine Variante davon, die sich von der Aminosäuresequenz von SEQ ID NO:2 durch Modifikation von mindestens einer der beiden N-terminalen Aminosäure unterscheidet. Das erfindungsgemäße Peptid, Polypeptid oder Protein bindet vorzugsweise an Endothelzellen der Lunge, insbesondere der menschlichen Lunge.

In einem weiteren Aspekt betrifft die Erfindung ein Peptid, Polypeptid oder Protein, das spezifisch an Endothelzellen der Lunge bindet, wobei das Peptid, Polypeptid oder Protein die Aminosäuresequenz von SEQ ID NO:3, SEQ ID NO:4 oder SEQ ID NO:5 umfasst.

Im Rahmen der vorliegenden Offenbarung bezeichnet der Begriff "Peptid" eine Verknüpfung von 2-10 Aminosäuren, die über eine peptidische Bindung miteinander verbunden sind. Der Begriff "Polypeptid" bezeichnet eine Verknüpfung von 11-100 Aminosäuren, die über eine peptidische Bindung miteinander verbunden sind. Polypeptide mit mehr als 100 Aminosäuren werden vorliegend als "Protein" bezeichnet.

In einer besonders bevorzugten Ausführungsform der Erfindung ist die erfindungsgemäße, lungenspezifische Peptidsequenz von SEQ ID NO:1 oder SEQ ID NO:2 oder der Variante von SEQ ID NO:2 Teil eines Kapsid-Proteins eines Virus. Dies bedeutet, dass die lungenspezifische Sequenz als Teil eines Kapsid-Proteins des Virus vorliegt. Um derartige Kapsid-Proteine herzustellen, wird eine entsprechende, für das lungenspezifische Peptid kodierende Nukleotidsequenz in die Region des Virus-Genoms kloniert, die für ein Kapsid-Protein des Virus kodiert. Wird die lungenspezifische Sequenz als Teil eines Kapsid-Proteins exprimiert, so kann sie vielfach an der Oberfläche des viralen Vektors präsentiert werden.

Vorzugsweise handelt es sich bei dem Kapsid-Protein um ein solches, das von einem Adeno-assoziierten Virus (AAV) stammt. Bei dem AAV kann es sich um einen beliebigen der im Stand der Technik beschriebenen Serotypen handeln, wobei das Kapsid-Protein vorzugsweise von einem AAV von einem der Serotypen 2, 4, 6, 8 und 9 stammt. Ein Kapsid-Protein eines AAV vom Serotyp 2 ist besonders bevorzugt.

Das Kapsid des AAV-Wildtyps setzt sich aus den Kapsid-Proteinen VP1, VP2 und VP3 zusammen, die überlappend von der cap-Region kodiert werden. Alle drei Proteine weisen einen identischen C-terminalen Bereich auf. Das Kapsid von AAV umfasst etwa 60 Kopien der Proteine VP1, VP2 und VP3, die im Verhältnis 1:1:8 exprimiert werden. Wird eine Nukleotidsequenz, die für eine der vorliegend beschriebenen lungenspezifischen Peptide kodiert, C-terminal in den Leserahmen von VP1 kloniert (d.h. in den Bereich, der bei allen drei Proteinen identisch ist), so kann man erwarten, dass theoretisch 60 der spezifischen Peptide der auf der Kapsid-Oberfläche zu finden sind.

Wird ein AAV-Vektor im Sinne der vorliegenden Erfindung modifiziert, so wird die für das lungenspezifische Peptid kodierende Nukleotidsequenz in die cap-Region am 3'-Ende des Genoms kloniert. Die für das lungenspezifische Peptid kodierende Sequenz kann in die genomische Sequenz von einem der Kapsid-Proteine VP1, VP2 oder VP3 kloniert werden. Die Kapsid-Proteine von AAV2 sind beispielhaft in SEQ ID NO:6 (VP1), SEQ ID NO:7 (VP2) und SEQ ID NO:8 (VP3) dargestellt.

In einer besonders bevorzugten Ausführungsform der Erfindung wird die für das lungenspezifische Peptid kodierende Sequenz in den Leserahmen eines VP1-Gens kloniert, vorzugsweise in das in SEQ ID NO:6 gezeigte VP1-Gen von AAV2. Dabei sollte beachtet werden, dass sich durch die Insertierung der klonierten Sequenz kein Wechsel des Leserahmens und kein vorzeitiger Abbruch der Translation ergibt. Dem Fachmann werden die dazu erforderlichen Methoden ohne Weiteres ersichtlich sein.

In allen drei Kapsid-Proteinen von AVV wurden Stellen identifiziert, an denen Peptidsequenzen für das Homing eingefügt werden können [15-20]. So wurde unter anderem das im VP1 von AAV2 vorkommende Arginin in Position 588 (R588) spezifisch für die Insertion eines Peptidliganden vorgeschlagen [21-22]. Diese Aminosäureposition des viralen Kapsids ist offenbar an der Bindung von AAV2 an seinen natürlichen Rezeptor beteiligt. Es wurde vermutet, dass R588 einer von vier Arginin-Resten ist, der die Bindung von AAV2 an seinen natürlichen Rezeptor vermittelt [23-24]. Eine Modifikation in diesem Bereich des Kapsids schwächt den natürlichen Tropismus von AAV2 ab oder beseitigt diesen vollständig.

Demgemäß ist es erfindungsgemäß besonders bevorzugt, dass die erfindungsgemäße lungenspezifische Peptidsequenz von SEQ ID NO:1 oder SEQ ID NO:2 (oder deren Variante) oder eine der lungenspezifischen Peptidsequenzen von SEQ ID NO:3, SEQ ID NO:4 oder SEQ ID NO:5 im Bereich der Aminosäuren 550-600 des VP1-Proteins von AAV2, insbesondere des Proteins von SEQ ID NO:6, insertiert vorliegt. Noch stärker bevorzugt ist es, die lungenspezifische Peptidsequenz im Bereich der Aminosäuren 560-600, 570-600, 560-590, 570-590 des VP1-Proteins inseriert vorliegen.

So kann sich die die Aminosäuresequenz von SEQ ID NO:1 oder SEQ ID NO:2 oder deren Variante (oder alternativ die Peptidsequenzen von SEQ ID NO:3, SEQ ID NO:4 oder SEQ ID NO:5) z.B. unmittelbar hinter einer der folgenden Aminosäure des VP1-Proteins, insbesondere des Proteins von SEQ ID NO:6, anschließen: 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599 oder 600. Besonders bevorzugt ist es, dass die Aminosäuresequenz von SEQ ID NO:1 oder SEQ ID NO:2 oder deren Variante (oder alternativ die Peptidsequenzen von SEQ ID NO:3, SEQ ID NO:4 oder SEQ ID NO:5) auf die Aminosäure 588 des VP1-Proteins von SEQ ID NO:6 folgt, wie es in den Beispielen gezeigt ist. Es ist dabei möglich, dass zwischen dem ArgininRest in Position 588 und der ersten Aminosäure von SEQ ID NO:1 oder SEQ ID NO:2 oder der Variante von SEQ ID NO:2 eine oder mehrere, und insbesondere bis zu 5 (d.h. 1, 2, 3, 4 oder 5) Aminosäuren liegen, die durch die Klonierung bedingt sind. Ebenso können hinter der letzten Aminosäure von SEQ ID NO:1 oder SEQ ID NO:2 oder der Variante von SEQ ID NO:2 eine oder mehrere, insbesondere bis zu 5 (d.h. 1, 2, 3, 4 oder 5) Aminosäuren liegen.

Die oben in Bezug auf VP1 angegebenen Stellen und Bereiche in der Aminosäuresequenz des Kapsid-Proteins gelten analog auch für die Kapsid-Proteine VP2 und VP3 von AAV2. Da sich die drei Kapsid-Proteine VP1, VP2 und VP3 von AAV2 lediglich durch die Länge der N-terminalen Sequenz unterscheiden und demgemäß einen identischen C-terminalen Bereich aufweisen, wird der Fachmann keine Probleme haben, durch Sequenzvergleich die oben für VP1 angegebenen Stellen für die Inserierung des Peptidliganden in den Aminosäuresequenzen von VP1 und VP2 zu identifizieren. So entspricht die Aminosäure R588 in VP1 der Position R451 von VP2 (SEQ ID NO:7) bzw. der Position R386 von VP3 (SEQ ID NO:8).

SEQ ID NO:9 zeigt beispielhaft die Sequenz des VP1-Proteins von AVV2 nach Einbringung der Peptidsequenz von SEQ ID NO:2. Bedingt durch die Klonierung weist das Kapsid-Protein zwei zusätzliche Aminosäuren auf, die in der nativen Sequenz des VP1-Proteins von AVV2 nicht vorkommen. So wird die Peptidsequenz von SEQ ID NO:2 N-terminal von einem Glycin in Position 589 und C-terminal von einem Alanin in Position 597 flankiert. Darüber hinaus ist das Asparagin in Position 587 der nativen Sequenz gegen ein Glutamin ausgetauscht.

In einer besonderen Ausführungsform betrifft die vorliegende Erfindung somit auch ein Kapsid-Protein, das Folgendes umfasst oder daraus besteht:
(a) die Aminosäuresequenz von SEQ ID NO:9;
(b) eine Aminosäuresequenz, die mindestens 80% Identität zu der Aminosäuresequenz von SEQ ID NO:9 aufweist; oder
(c) ein Fragment von einer der in (a) oder (b) definierten Aminosäuresequenzen.

In einem weiteren Aspekt richtet sich die Erfindung auf ein virales Kapsid, das ein Peptid, Polypeptid oder Protein umfasst, welches spezifisch an Zellen der Lunge bindet und die Aminosäuresequenz von SEQ ID NO:1 oder SEQ ID NO:2 oder einer wie oben beschriebene Variante von SEQ ID NO:2 (oder alternativ die Peptidsequenzen von SEQ ID NO:3, SEQ ID NO:4 oder SEQ ID NO:5) umfasst.

Des Weiteren stellt die vorliegende Erfindung auch eine Nukleinsäure bereit, die für ein wie oben beschriebenes Peptid, Polypeptid oder Protein kodiert. Eine Nukleinsäure, die für ein Kapsid-Protein kodiert, welches ein wie oben beschriebenes, erfindungsgemäßes Peptid, Polypeptid oder Protein umfasst, wird ebenfalls bereitgestellt. Vorzugsweise umfasst die für das Kapsid-Protein kodierende Nukleinsäure die Nukleotidsequenz von SEQ ID NO:10 oder eine davon abgeleitete Nukleotidsequenz mit mindestens 80% Sequenzidentität. Ein Plasmid, das eine solche Nukleinsäure umfasst, wird ebenfalls bereitgestellt.

In einem noch weiteren Aspekt betrifft die Erfindung einen rekombinanten, d.h. einen mittels gentechnischer Verfahren hergestellten, viralen Vektor, der ein Kapsid und mindestens ein darin verpacktes Transgen umfasst, wobei das Kapsid mindestens ein Kapsid-Protein umfasst, welches die Aminosäuresequenz von SEQ ID NO:1 oder SEQ ID NO:2 oder von einer Variante von SEQ ID NO:2 umfasst, die sich von der Aminosäuresequenz von SEQ ID NO:2 durch Modifikation von mindestens einer der beiden N-terminalen Aminosäure unterscheidet. Alternativ kann das Kapsid-Protein auch die Aminosäuresequenz von SEQ ID NO:3, SEQ ID NO:4 oder SEQ ID NO:5 umfassen. Bei dem rekombinanten viralen Vektor kann es sich um einen rekombinanten AAV-Vektor handeln, z.B. um einen solchen vom Serotyp 2, 4, 6, 8 und 9. AAV-Vektoren vom Serotyp 2 sind besonders bevorzugt.

Die unterschiedlichen AAV-Serotypen unterscheiden sich im Wesentlichen anhand ihres natürlichen Tropismus. So bindet Wildtyp-AAV2 stärker an alveolare Zellen als an Lungenepithelzellen, während AAV5, AAV6 und AAV9 zu einer stärkeren Transduktion der epithelialen Zellen der Atemwege in der Lage sind. Der Fachmann kann diese natürlichen Unterschiede hinsichtlich der Spezifität der Zellen nutzen, um die durch die erfindungsgemäßen Peptide vermittelte Spezifität für bestimmte Zellen oder Gewebe weiter zu verstärken. Auf der Nukleinsäureebene sind die verschiedenen AAV-Serotypen stark homolog. So sind z.B. die Serotypen AAV1, AAV2, AAV3 und AAV6 auf der Nukleinsäureebene zu 82% identisch [25].

Das Kapsid der erfindungsgemäßen viralen Vektoren umfasst ein oder mehrere Transgene. Als Transgen wird vorliegend ein Gen bezeichnet, das mittels gentechnischer Verfahren in das Genom des Vektors eingebracht wurde. Bei dem Transgen (oder den Transgenen) kann es sich um DNA oder RNA handeln. Vorzugsweise handelt es sich um einzelsträngige DNA (ssDNA) oder doppelsträngige DNA (dsDNA), wie beispielsweise genomische DNA oder cDNA. Vorzugsweise handelt es sich bei dem Transgen, welches mit Hilfe des erfindungsgemäßen rekombinanten viralen Vektors transportiert werden soll, um ein humanes Gen. Geeignete Transgene sind z.B. therapeutische Gene, die ein im Patienten dysfunktionales Gen ersetzen sollen. Es kann sich ferner um ein Gen handeln, das in dem entsprechenden Zielgewebe nicht oder nur in unzureichendem Umfang exprimiert wird. In einer bevorzugten Ausführungsform handelt es sich bei dem Transgen, welches vom viralen Vektor der Erfindung exprimiert wird, um ein Gen, das für eine Stickstoffmonoxid-Synthase (NOS) oder für den Bone-Morphogenic-Protein-Rezeptor 2 (BMPR2) kodiert. Bei der NOS kann es sich um eine endotheliale NOS (eNOS) oder eine induzierbare NOS (iNOS) kodiert. Die für eNOS und iNOS kodierenden Gene können für die Behandlung einer Lungenfunktionsstörung oder Lungenerkrankung, insbesondere der pulmonalen oder pulmonal-arteriellen Hypertonie, in einem Subjekt eingesetzt werden.

In einer weiteren Ausführungsform kodiert das Transgen für ein radioprotektives Protein, wie z.B. für ein radioprotektives Enzym. Eine wesentliche Limitation beim Einsatz der Radiotherapie im Rahmen der Behandlung von Krebs ist die strahleninduzierte Schädigung des Normalgewebes, was häufig eine Verringerung der Strahlendosis, eine Unterbrechung des Therapieregimes, oder sogar einen völligen Verzicht auf diese Therapieform nötig macht. Die Lunge ist ein therapeutisch besonders relevanter Ort des Tumorwachstums aber gleichzeitig ein besonders strahlenempfindliches Organ, weshalb Primärtumoren dort oft nur eingeschränkt und diffuses Tumorwachstum meistens gar nicht radiotherapeutisch behandelt werden kann. Mit Hilfe der lungenspezifischen Vektoren der vorliegenden Erfindung kann das gesunde Gewebe, welches das maligne Gewebe umgibt, durch zielgerichtete Expression radioprotektiver Proteine geschützt werden. In einer bevorzugten Ausführungsform handelt es sich bei dem Transgen, welches in das gesunde Lungengewebe eingeschleust wird, um eine Mangan-Superoxiddismutase (MnSOD), die die Konversion von Superoxidanionen, einem der wesentliche Faktoren der strahleninduzierten Toxizität, zu Wasserstoffperoxid katalysiert. In einer weiteren hier vorgeschlagenen Ausführungsform handelt es sich um die Kinasedomäne des Ataxia telangiectasia mutant (ATM)-Gens, das zur Reparatur der durch Bestrahlung entstandenen DNA-Schäden beiträgt. In einer weiteren bevorzugten Ausführungsform werden beide Gene mittels der vorliegend beschriebenen Vektoren in den zu behandelnden Patienten eingebracht.

In einer weiteren Ausführungsform kodiert das Transgen für humanes Alpha-l-Antitrypsin. Der Mangel an ausreichenden Mengen Alpha-1-Antitrypsin ist ursächlich für das Laurell-Eriksson-Syndrom, eine erbliche Stoffwechselerkrankung, die zu einem Lungenemphysem oder einer Leberzirrhose führen kann. Alpha-1-Antitrypsin ist für die Regulation der Aktivität von Proteasen im Serum erforderlich. Der Mangel dieses Inhibitors führt zu einer verstärkten Proteolyse im Serum und dadurch bedingt zu den genannten, schwerwiegenden Folgeerscheinungen.

Die viralen Vektoren der vorliegenden Erfindung eignen sich insbesondere zur Verwendung in einem Verfahren zur therapeutischen Behandlung von Erkrankungen der Lunge. Erkrankungen der Lunge im Sinne der Erfindung umfassen insbesondere alle Arten von Gefäßerkrankungen der Lunge, wie die pulmonale Hypertonie, aber auch Lungentumore, Alpha-1-Antitrypsin-Mangel (A1AD), und andere. Für die Behandlung mit den erfindungsgemäßen Vektoren geeignete Lungentumoren umfassen beispielsweise das kleinzellige Bronchialkarzinom (SCLC), das Plattenepithelkarzinom, das Adenokarzinom und das großzellige Bronchialkarzinom. In einer bevorzugten Ausführungsform werden die viralen Vektoren der vorliegenden Erfindung zur therapeutischen Behandlung der der pulmonalen oder pulmonal-arteriellen Hypertonie eingesetzt.

In einer noch weiteren Ausführungsform kodiert das Transgen für ein Antitumormittel, wie z.B. ein Tumorsupressor-Protein, oder einen Immunmodulator, wie z.B. ein Zytokin (z.B. Interleukin 1 bis 4, Gamma-Interferon, p53), das selektiv zum Lungengewebe des Patienten transportiert werden soll.

Die Vektoren können ferner auch dazu verwendet werden, um Antisense-RNA, Ribozyme, oder Ähnliches in das Endothelgewebe der Lunge zu transportieren. Ferner können die erfindungsgemäßen Vektoren auch Transgene umfassen, die für sekretorische Proteine kodieren, die systemisch in die Blutlaufbahn gelangen sollen. Solche sekretorischen Proteine können durch das Lungenkapillarbett, welches Teil des Herzkreislaufsystems ist, effizient in die Blutbahn abgeschieden werden.

Wie vorliegend verwendet, bezeichnet der Begriff "Subjekt" alle menschlichen oder tierischen Organismen, die durch AAV-Vektoren infiziert werden können. Vorzugsweise handelt es sich bei dem zu behandelnden Subjekt um ein Säugetier, wie z.B. um einen Menschen, einen Primaten, eine Maus oder eine Ratte. In einer bevorzugten Ausführungsform ist das zu behandelnde Subjekt ein Mensch. Nach Transfektion in das Subjekt sorgt der Vektor für die ortsspezifische Expression des Transgens in den Zellen des Lungenendothels.

Das Transgen kann in dem viralen Vektor in Form einer Expressionskassette vorliegen, die neben der zu exprimierenden Sequenz des Transgens weitere für die Expression erforderliche Elemente umfasst, wie z.B. einen geeigneten Promoter, der nach Infektion der entsprechenden Zellen die Expression des Transgens steuert. Geeignete Promotoren umfassen neben den AAV-Promotoren z.B. den Cytomegalovirus (CMV)-Promoter, den Chicken-Beta-Actin/Cytomegalovirus-Hybridpromoter (CAG), einen Endothelzellen-spezifischen Promoter, wie z.B. den VE-Catherin Promoter, sowie Steroid-Promotoren und Metallothionein-Promotoren. In einer besonders bevorzugten Ausführungsform umfasst das erfindungsgemäße Transgen einen Lungenendothelspezifischen Promoter, der in funktionaler Verbindung mit dem zu exprimierenden Transgen verbunden ist. Auf diese Weise kann die Spezifität der erfindungsgemäßen Vektoren für Lungenendothelzellen weiter erhöht werden. Wie hierin verwendet, ist ein Lungenendothel-spezifischer Promoter ein Promoter, dessen Aktivität in Lungenendothelzellen mindestens 2-fach, 5-fach, 10-fach, 20-fach, 50-fach oder 100-fach höher ist als in einer Zelle, die keine Lungenendothelzelle ist. Vorzugsweise ist dieser Promoter ein humaner Promoter. Die Expressionskassette kann auch ein Enhancer-Element zur Erhöhung des Expressionslevels des zu exprimierenden exogenen Proteins enthalten. Ferner kann die Expressionskassette Polyadenylierungssequenzen, wie z.B. die SV40-Polyadenylierungssequenzen oder die Polyadenylierungssequenzen des bovinen Wachstumshormons umfassen.

Die erfindungsgemäßen viralen Vektoren können, vorzugsweise als Teile eines ihrer Kapsid-Proteine, die Aminosäuresequenz von SEQ ID NO:1, SEQ ID NO:2, SEQ ID NO:3, SEQ ID NO:4 oder SEQ ID NO:5 umfassen. Alternativ können auch Varianten der Aminosäuresequenz von SEQ ID NO:2 verwendet werden, die sich von der Aminosäuresequenz von SEQ ID NO:2 durch Modifikation von mindestens einer der beiden N-terminalen Aminosäure unterscheiden. Bei der Modifikation kann es sich um einen Austausch, eine Deletion oder eine Insertion von Aminosäuren handeln, solang die Variante die Fähigkeit beibehält, als Teil des Kapsids die spezifische Bindung des Vektors an Rezeptor-Strukturen von Endothelzellen der Lunge zu vermitteln. Die Erfindung erstreckt sich somit auch auf Varianten der Sequenz von SEQ ID NO:2, bei denen eine der beiden N-terminalen Aminosäuren von SEQ ID NO:2 verändert wurde. Diese Varianten, die vom Umfang der Erfindung umfasst werden, weisen folglich eine Sequenzidentität von mehr als 85% zu der in SEQ ID NO:2 gezeigten Aminosäuresequenz auf, wenn die Sequenzen mit den Programmen GAP oder BESTFIT miteinander verglichen werden. Diese Computerprogramme zur Bestimmung der Aminosäuresequenzidentität sind im Stand der Technik hinreichend bekannt.

Die Variante der Sequenz von SEQ ID NO:2 kann auf einer Substitution von einer oder von beiden N-terminalen Aminosäuren beruhen, d.h. es können eine oder beide N-terminalen Aminosäuren gegen eine andere Aminosäure ausgetauscht sein. Vorzugsweise ist die Substitution, durch die sich die Varianten von der in SEQ ID NO:2 dargestellten Aminosäuresequenz unterscheidet, eine konservative Substitution, d.h. eine Substitution einer Aminosäure durch eine Aminosäure ähnlicher Polarität, die dem Peptid ähnliche funktionelle Eigenschaften verleiht. Vorzugsweise stammt die ausgetauschte Aminosäure aus der gleichen Gruppe von Aminosäuren wie die ersetzende Aminosäuren. Beispielsweise kann ein hydrophober Rest durch einen anderen hydrophoben Rest ersetzt werden oder ein polarer Rest durch einen anderen polaren Rest. Funktionell ähnliche Aminosäuren, die im Rahmen einer konservativen Substitution gegeneinander ausgetauscht werden können, umfassen z.B. nicht-polare Aminosäuren wie z.B. Glycin, Valin, Alanin, Isoleucin, Leucin, Methionin, Prolin, Phenylalanin, und Tryptophan. Beispiele von ungeladenen polaren Aminosäuren umfassen Serin, Threonin, Glutamin, Asparagin, Tyrosin, und Cystein. Beispiele von geladenen polaren (sauren) Aminosäuren umfassen Histidin, Arginin und Lysin. Beispiele von geladenen polaren (basischen) Aminosäuren umfassen Asparaginsäure und Glutaminsäure.

Als Varianten der in SEQ ID NO:2 gezeigten Aminosäuresequenz gelten auch solche Aminosäuresequenzen, bei denen eine Aminosäure im Bereich der beiden N-terminalen Aminosäuren von SEQ ID NO:2 eingefügt wurde. Solche Insertionen können prinzipiell erfolgen, so lange die resultierende Variante ihre Fähigkeit beibehält, spezifisch an Endothelzellen der Lunge zu binden. Ferner gelten vorliegend auch solche Proteine als Varianten der in SEQ ID NO:2 gezeigten Aminosäuresequenz, bei denen eine der beiden N-terminalen Aminosäuren von SEQ ID NO:2 fehlt. Voraussetzung ist wiederum, dass die entsprechend deletierte Variante spezifisch an Endothelzellen der Lunge bindet.

Ebenfalls von der Erfindung umfasst sind Lungenendothelspezifische Varianten der in SEQ ID NO:2 gezeigten Aminosäuresequenz, die an einer oder an beiden N-terminalen Aminosäuren strukturell modifiziert sind, wie beispielsweise durch Einführen einer modifizierten Aminosäure. Bei diesen modifizierten Aminosäuren kann es sich erfindungsgemäß um Aminosäuren handeln, die durch Biotinylierung, Phosphorylierung, Glykosylierung, Acetylierung, Verzweigung und/oder Zyklisierung verändert wurden.

Virale Vektoren, deren Kapsid eine der erfindungsgemäßen Peptidsequenzen oder eine wie oben definierte Variante davon umfassen, binden spezifisch an Endothelzellen der Lunge. Wie vorliegend verwendet bedeutet eine "spezifische" Bindung der erfindungsgemäßen Vektoren, dass sich die Vektoren nach systemischer Verabreichung vorwiegend an oder in den Endothelzellen der Lunge anreichern. Dies bedeutet, dass sich mehr als 50% der ursprünglich verabreichten Vektorgenome in den Endothelzellen oder im Bereich der Endothelzellen der Lunge anreichern, während sich weniger als 50% in oder im Bereich von anderen Zellen oder Geweben anreichern (wie z.B. in der Milz oder Leer). Es ist bevorzugt, dass sich mehr als 60%, 70%, 80%, 90%, 95%, oder sogar mehr als 99% der ursprünglich verabreichten Vektorgenome in oder im Bereich der Endothelzellen der Lunge anreichern. Eine spezifische Bindung der Vektoren kann auch über die Expression des Transgens ermittelt werden. Bei viralen Vektoren, die spezifisch an Endothelzellen der Lunge binden, erfolgt mehr als 50% der gesamten Expression des Transgens in oder im Bereich der Endothelzellen der Lunge, während weniger als 50% der Expression in oder im Bereich von anderen Geweben beobachtet werden kann. Es ist allerdings bevorzugt, dass mehr als 60%, 70%, 80%, 90%, 95%, oder sogar mehr als 99% der insgesamt gemessenen Expression des Transgens in oder im Bereich der Endothelzellen der Lunge erfolgt.

Der Fachmann wird problemlos in der Lage sein, die spezifische Bindung der erfindungsgemäßen Vektoren an Endothelzellen der Lunge und die Expression des mit Hilfe der Vektoren eingeschleusten Transgens zu bestimmten. Zu diesem Zweck geeignete Verfahren zur Messung der Spezifität von Transduktion und Expression sind in den nachstehenden Beispielen gezeigt.

Es ist darüber hinaus erfindungsgemäß bevorzugt, dass Vektoren, deren Kapside Varianten der in SEQ ID NO:2 gezeigten Aminosäuresequenz enthalten, mindestens etwa 50% der Bindungsaktivität eines entsprechenden viralen Vektors aufweisen, dessen Kapsid die in SEQ ID NO:2 gezeigte Aminosäuresequenz aufweist. Noch stärker bevorzugt ist es, dass die Varianten etwa 60%, 70%, 80%, 90%, 95%, 96%, 97%, 98% oder 99% der Bindungsaktivität eines entsprechenden viralen Vektors aufweisen, dessen Kapsid die in SEQ ID NO:2 gezeigte Aminosäuresequenz aufweist. Die Bindungsaktivität der Vektoren kann mit Hilfe von in vitro-Assays gemessen werden, wie sie in den vorliegenden Beispielen beschrieben werden.

Vorzugsweise ist die Bindungsaktivität der erfindungsgemäßen Vektoren, wie sie durch Verteilung der Vektorgenome oder Expression des Transgens bestimmt werden kann, für Endothelzellen der Lunge mindestens 2-fach, 5-fach, 10-fach, 20-fach, 50-fach, 75-fach, 100-fach, 150-fach, 200-fach, 250-fach, 500-fach, 600-fach, 700-fach, 800-fach, 900-fach, 1000-fach, 2000-fach, 5000-fach, oder 10000-fach höher als die Bindungsaktivität für eine Kontroll-Zelle, die keine Lungenendothelzelle ist, Z.B. eine Milz- oder Leber-Zelle).

Die Erfindung betrifft auch eine Zelle, die ein erfindungsgemäßes Peptid, Polypeptid oder Protein, eine dafür kodierende Nukleinsäure, ein Plasmid, welches eine solche Nukleinsäure umfasst, oder einen wie oben beschriebenen rekombinanten AAV-Vektor enthält. Es handelt sich vorzugsweise um eine humane Zelle oder Zelllinie.

In einer Ausführungsform wurde eine Zelle z.B. durch ein Biopsieverfahren aus einem humanen Subjekt gewonnen und anschließend mit dem viralen Vektor in einem ex *vivo* Verfahren transfiziert. Die Zelle kann anschließend dem Subjekt reimplantiert oder diesem auf andere Weise wieder zugeführt werden, z.B. durch Transplantation oder Infusion. Die Wahrscheinlichkeit einer Abstoßung von transplantierten Zellen ist geringer, wenn das Subjekt, aus dem die Zelle gewonnen wurde, genetisch dem Subjekt ähnelt, an das die Zelle verabreicht wird. Vorzugsweise handelt es sich daher bei dem Subjekt, dem die transfizierten Zellen zugeführt werden, um dasselbe Subjekt, aus dem zuvor die Zellen gewonnen wurden. Die Zelle ist vorzugsweise eine humane Lungenzelle, insbesondere eine Zelle des humanen Lungenendothels. Die die zu transfizierende Zelle kann auch eine Stammzelle, wie z.B. eine adulte humane Stammzelle, sein. Es ist erfindungsgemäß besonders bevorzugt, dass es sich bei den zu transfizierenden Zellen um autologen Zellen handelt, die mit dem erfindungsgemäßen viralen Vektor, z.B. dem beschriebenen rekombinanten AAV2-Vektor, *ex vivo* transfiziert wurde. Die Zellen werden vorzugsweise in einem Verfahren zur Behandlung einer Lungenfunktionsstörung oder einer Erkrankung der Lunge in einem Subjekt verwendet.

In einem weiteren Aspekt betrifft die Erfindung ein Verfahren zur Herstellung eines AAV-Vektors, bei dem ein Plasmid verwendet wird, dass für ein Kapsid-Protein kodiert, welches die Aminosäuresequenz von SEQ ID NO:1 oder SEQ ID NO:2 oder einer Variante davon umfasst. Alternativ kann der Vektor auch die Aminosäuresequenz von SEQ ID NO:3, SEQ ID NO:4 oder SEQ ID NO:5 umfassen. Das grundsätzliche Verfahren der Herstellung von rekombinanten AAV-Vektoren, die ein zu exprimierendes Transgen umfassen, ist im Stand der Technik hinreichend beschrieben [28]. HEK 293-T Zellen werden mit drei Plasmiden transfiziert. Ein erstes Plasmid enthält das die cap- und rep-Regionen des AAV-Genoms, wobei jedoch die natürlich vorkommenden Inverted Repeats (ITRs) fehlen. Die cap-Region dieses Plasmids enthält ein Gen, das für mindestens ein modifiziertes Kapsid-Protein kodiert, d.h. ein Gen, welches die erfindungsgemäße Peptidsequenz umfasst. Ein zweites Plasmid umfasst eine Transgen-Expressionskassette, die von den entsprechenden ITRs flankiert wird, die das Verpackungssignal darstellen. Die Expressionskassette wird daher im Zuge des Zusammenbaus der Viruspartikel in das Kapsid verpackt. Bei dem dritten Plasmid handelt es sich um ein adenovirales Helferplasmid, auf dem die Helferproteine E1A, E1B, E2A, E4-orf6, VA kodiert sind, die für die AAV-Replikation in den HEK 293-T-Zellen erforderlich sind.

Bedingungen, welche die Anreicherung und Aufreinigung der erfindungsgemäßen rekombinanten Vektoren erlauben, sind im Stand der Technik bekannt. Die erfindungsgemäßen Vektoren können beispielsweise durch Gelfiltrationsverfahren, z.B. unter Verwendung einer Sepharosematrix, entsalzt und durch anschließende Filtration aufgereinigt werden. Andere Aufreinigungsverfahren können ferner eine Cäsiumchlorid- oder Iodixanol-Gradienten-Ultrazentrifugation umfassen. Die Aufreinigung reduziert potentiell abträgliche Affekte in dem Subjekt, an das die adeno-assoziierten viralen Vektoren verabreicht werden. Der verabreichte Virus ist im Wesentlichen frei von Wildtyp- und replikationskompetenten Virus. Die Reinheit des Virus kann durch geeignete Verfahren, wie PCR-Amplifikation, überprüft werden.

Die Erfindung stellt in einem weiteren Aspekt eine pharmazeutische Zusammensetzung bereit, die einen viralen Vektor der vorliegenden Erfindung, insbesondere einen AAV-Vektor, umfasst. Der virale Vektor wird dabei in einer therapeutisch wirksamen Menge an den Patienten verabreicht, d.h. in einer Menge, die ausreicht, um mindestens ein Symptom der zu behandelnden Lungenfunktionsstörung oder Lungenerkrankung in einem erheblichen Maße zu verbessern oder die Progression der Erkrankung zu unterbinden. Symptome, die regelmäßig mit einer Lungenerkrankung verbunden sind, sind z.B. Husten, Fieber, Schmerzen des Brustkorbes, Heiserkeit und Atemschwierigkeiten. Eine therapeutisch wirksame Menge des erfindungsgemäßen Vektors ruft eine positive Veränderung in einem der besagten Symptom hervor, d.h. eine Veränderung die den Phänotyp des betroffenen Subjekts an den Phänotyp eines gesunden Subjekts, das nicht unter einer Lungenerkrankung leidet, annähert.

In einer bevorzugten Ausführungsform der Erfindung erfolgt die Verabreichung des viralen Vektors in einer Menge, die zu einer vollständigen oder im Wesentlichen vollständigen Heilung der Lungenfunktionsstörung oder Lungenerkrankung führt. Die pharmazeutische Zusammensetzung wird demgemäß eine therapeutisch wirksame Dosis des erfindungsgemäßen Vektors enthalten. Eine therapeutisch wirksame Dosis wird in der Regel für das Subjekt, welches sich der Behandlung unterzieht, nicht toxisch sein.

Die genaue Menge an viralen Vektor, die verabreicht werden muss, um einen therapeutischen Effekt zu erzielen, hängt von mehreren Parametern ab. Faktoren, die relevant für die Menge des zu verabreichenden viralen Vektors sind, umfassen z.B. die Art der Verabreichung des viralen Vektors, die Art und Schwere der Lungenerkrankung, die Krankheitsgeschichte des zu behandelnden Patienten sowie Alter, Gewicht, Größe und Gesundheitszustand des zu behandelnden Patienten. Ferner sind auch das Expressionslevel des Transgens, das benötigt wird um einen therapeutischen Effekt zu erzielen, die Immunantwort des Patienten, sowie die Stabilität des Genproduktes für die zu verabreichende Menge relevant. Eine therapeutisch wirksame Menge des viralen Vektors kann von einem Fachmann ohne Weiteres auf Basis des allgemeinen Fachwissens und der vorliegenden Offenbarung bestimmt werden.

Der virale Vektor wird vorzugsweise in einer Menge verabreicht, die einer Virusdosis im Bereich von 1,0 x 10¹⁰ bis 1,0 x 10¹⁴ vg/kg (Virusgenome pro kg Körpergewicht) entspricht, wobei ein Bereich von 1,0 x 10¹¹ bis 1,0 x 10¹³ vg/kg stärker bevorzugt ist, und ein Bereich von 5,0 x 10¹¹ bis 5,0 x 10¹² vg/kg stärker bevorzugt ist, und ein Bereich von 1,0 x 10¹² bis 5,0 x 10¹² noch stärker bevorzugt ist. Eine Virusdosis von etwa 2,5 x 10¹² vg/kg ist am stärksten bevorzugt. Die zu verabreichende Menge des viralen Vektors, z.B. des erfindungsgemäßen AAV2-Vektors, kann abhängig von der Stärke der Expression des einen oder der mehreren Transgene angepasst werden.

Der virale Vektor der vorliegenden Erfindung, wie z.B. der erfindungsgemäß bevorzugte AVV2-Vektor, kann für verschiedene Arten der Verabreichung formuliert werden, z.B. für die orale Verabreichung als Kapsel, als Flüssigkeit oder Ähnliches. Es ist jedoch bevorzugt, dass der virale Vektor parenteral, vorzugsweise mittels intravenöser Injektion oder intravenöser Infusion verabreicht wird. Die Verabreichung kann beispielsweise mittels intravenöser Infusion, z.B. innerhalb von 60 Minuten, innerhalb von 30 Minuten, oder innerhalb von 15 Minuten erfolgen. Es ist ferner bevorzugt, dass der virale Vektor während einer Operation an der Lunge lokal mittels Injektion verabreicht wird. Zusammensetzungen, die für die Verabreichung mittels Injektion und/oder Infusion geeignet sind, umfassen in der Regel Lösungen und Dispersionen sowie Pulver, aus denen entsprechende Lösungen und Dispersionen hergestellt werden können. Derartige Zusammensetzungen werden den viralen Vektor und mindestens einen geeigneten pharmazeutisch akzeptablen Träger enthalten. Geeignete pharmazeutisch akzeptable Träger für die intravenöse Verabreichung umfassen bakteriostatisches Wasser, Ringer-Lösung, physiologische Salzlösung, Phosphatgepufferte Salzlösung (PBS) und Cremophor EL™. Sterile Zusammensetzungen für die Injektion und/oder Infusion können hergestellt werden, indem der viralen Vektor in der erforderlichen Menge in einem geeigneten Träger eingebracht und anschließend mittels eines Filters sterilfiltriert wird. Zusammensetzungen für die Verabreichung mittels Injektion oder Infusion sollten nach ihrer Herstellung unter Lagerungsbedingungen über einen längeren Zeitraum stabil bleiben. Die Zusammensetzungen können zu diesem Zweck ein Konservierungsmittel enthalten. Geeignete Konservierungsmittel umfassen Chlorbutanol, Phenol, Ascorbinsäure und Thiomersal. Die Herstellung von entsprechenden Darreichungsformen sowie geeignete Hilfsstoffe sind beispielsweise in "Remington: The Science and Practice of Pharmacy", Lippincott Williams & Wilkins; 21. Auflage (2005) beschrieben.

In noch einem weiteren Aspekt betrifft die Erfindung ein Verfahren zur therapeutischen Behandlung einer Lungenfunktionsstörung oder einer Lungenerkrankung, bei dem man einen erfindungsgemäßen viralen Vektor, vorzugsweise einen AAV-Vektor, wie er oben beschrieben ist, an ein Subjekt verabreicht. Der Vektor umfasst ein Kapsid, das mindestens ein Kapsid-Protein umfasst, welches die Aminosäuresequenz von SEQ ID NO:1 oder SEQ ID NO:2 oder eine wie oben beschriebene Variante von SEQ ID NO:2 enthält. Alternativ kann der Vektor auch die Aminosäuresequenz von SEQ ID NO:3, SEQ ID NO:4 oder SEQ ID NO:5 umfassen. Der virale Vektor umfasst ferner ein Transgen, z.B. ein therapeutisches Gen, das für die Behandlung der Lungenfunktionsstörung oder Lungenerkrankung nützlich ist. Nach Verabreichung an das zu behandelnde Subjekt, vorzugsweise durch systemische Gabe, wie z.B. intravenöse Injektion oder Infusion, sorgt der Vektor für die spezifische Expression des Gens in den Endothelzellen der Lunge.

### KURZE BESCHREIBUNG DER FIGUREN

**Figur 1** zeigt das im Rahmen der Erfindung angewendete *in vivo* Selektionsverfahren der AAV-Peptidbibliothek.
**Figur 2** zeigt die Sequenzen der mittels des Selektionsverfahrens identifizierten lungenspezifischen Peptide. Nach der vierten Selektionsrunde konnten insgesamt vier verschiedene Sequenzen identifiziert werden.

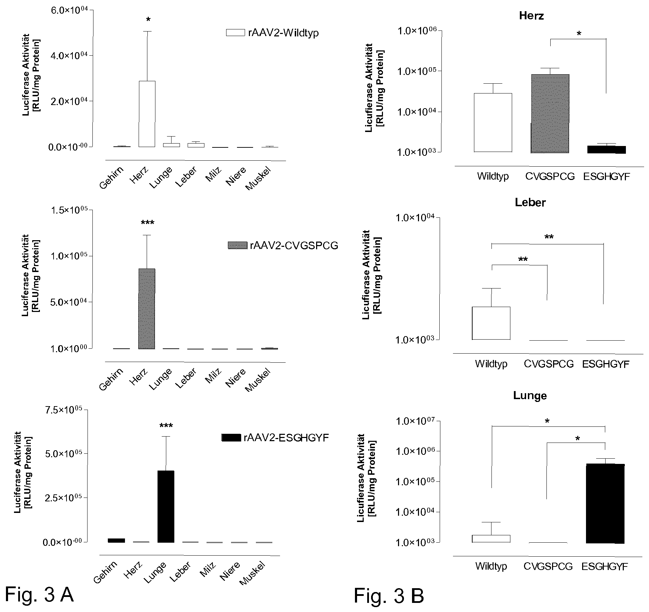

**Figur 3** zeigt die Messung der Expression von Luziferase 14 Tage nach systemischer Verabreichung rekombinanter AAV-Vektoren in Organlysaten der Maus. A: AAV2-Wildtyp-Vektor (obere Tafel) und die Insertionskontrolle AAV2-CVGSPCG (mittlerer Tafel) induzieren eine überwiegend herzspezifische Expression. AAV2-ESGHYGF (untere Tafel) induziert eine starke und zugleich lungenspezifische Expression der Luziferase. B: Vergleich der Expressionsstärke der Vektoren AAV2-Wildtyp, AAV2-CVGSPCG und AAV2-ESGHYGF in Herz (obere Tafel), Leber (mittlere Tafel) und Lunge (untere Tafel). AAV2-ESGHYGF zeigt eine stark abgeschwächte Expressionsinduktion in Herz und Leber und eine signifikante Erhöhung der Expression von Luziferase in der Lunge. Gezeigt sind Mittelwerte mit Standardabweichung. One-Way ANOVA. p<0,05=*; p<0,01=**; p<0,001=*** für n=3.
**Figur 4** zeigt eine Langzeit-Expressionsanalyse in der Maus nach systemischer Gabe von rekombinantem AAV2-ESGHYGF Vektor. Wiederholte Messungen mittels IVIS® 200 Imaging System zeigen eine stabile Genexpression in der Lunge über einen Zeitraum von 168 Tagen (n=1).

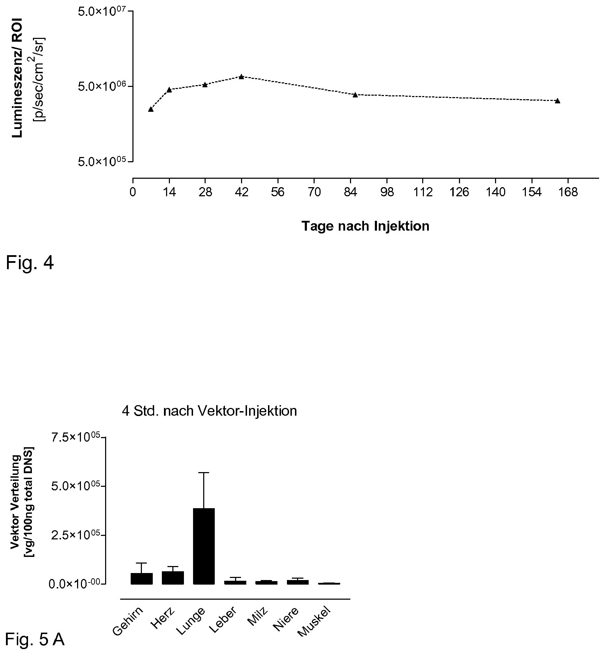

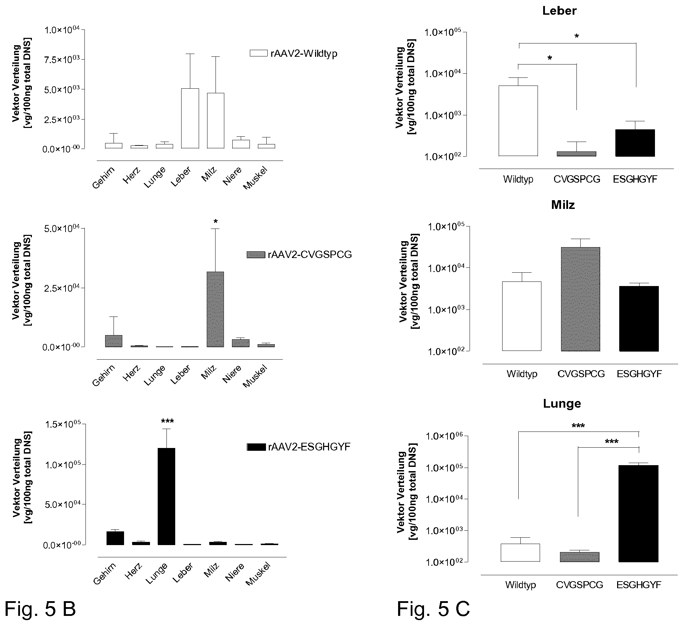

**Figur 5** zeigt die Verteilung rekombinanter AAV-Vektoren nach systemischer Verabreichung von 5x10¹⁰ gp/Maus mittels quantitativer Real-Time-PCR. A: Verteilung von AAV2-ESGHYGF 4 Stunden nach Vektorgabe in sieben verschiedenen Organen. B: Verteilung von Genomen, die durch den AAV2-Wildtyp (obere Tafel), den Kontrollvektor AAV2-CVGSPCG (mittlere Tafel) und AAV2-ESGHYGF (untere Tafel) bereitgestellt wurden. Kontrollvektor und Wildtyp reichern sich im retikulo-endothelialen System der Leber und der Milz an. AAV2-ESGHYGF akkumuliert ausschließlich in der Lunge. B: Vergleich der Verteilung von AAV2-Wildtyp, Kontrollvektor AAV2-CVGSPCG und AAV2-ESGHYGF in Leber (obere Tafel), Milz (mittlere Tafel) und Lunge (untere Tafel). Gezeigt sind Mittelwerte mit Standardabweichung. One-Way ANOVA. p<0,05=*; p<0,01=**; p<0,001=*** für n=3.

### BEISPIELE

Alle Daten wurden als Mittelwerte + Standardabweichung (SD) bestimmt. Die statistische Analyse wurde unter Verwendung des GraphPad Prism Programms 3.0 (GraphPad Software, San Diego, USA) durchgeführt. Die Daten wurden durch Einweg-Anova, gefolgt von multiplen Vergleichstests nach Bonferoni analysiert. P-Werte >0,05 wurden als signifikant erachtet.

### Beispiel 1: Selektion von AAV2-Peptidbibliotheken

Um gewebespezifische AAv2-Kapside zu selektieren, wurde eine Zufalls-Display-Peptidbibliothek hergestellt und über vier Runden selektiert. Eine zufallsbasierte X₇-AAV-Peptidbibliothek mit einer theoretischen Diversität von 1x10⁸ einzeln vorkommenden Klonen wurde unter Verwendung eines zweistufigen Protokolls hergestellt, wie es zuvor beschrieben wurde [26-27]. Es wurde zunächst ein degeneriertes Oligonukleotid hergestellt, das für sieben zufällig angeordnete Aminosäuren an der Nukleotidposition 3967 im AAV-Genom kodiert, was der Aminosäureposition R588 in VP1 entspricht. Das Oligonukleotid hatte die Sequenz: 5' - CAGTCGGCCAGAGAGGC (NNK) ₇GCCCAGGCGGCTGACGAG - 3' (SEQ ID NO:11). Der zweite Strang wurde unter Verwendung einer Sequenase (Amersham, Freiburg, Deutschland) und des Primers mit der Sequenz 5'-CTCGTCAGCCGCCTGG-3' (SEQ ID NO:12) hergestellt. Das doppelsträngige Insert wurde mit BglI geschnitten, mit dem QIAquick Nucleotide Removal Kit (Qiagen, Hilden, Deutschland) aufgereinigt und in das mit SfiI verdaute Bibliothek-Plasmid pMT187-0-3 ligiert [26]. Die Diversität des Plasmid-Bibliothek wurde anhand der Anzahl von Klonen bestimmt, die ausgehend von einem repräsentativen Aliquot von transformierten, elektrokompetenten DH5α-Bakterien auf Agar wuchsen, der 150 mg/ml Ampicillin enthielt. Bibliothek-Plasmide wurden geerntet und unter Verwendung des Plasmid Preparation Kit von Qiagen aufgereinigt. Die AAV-Bibliothek-Genome wurden in chimäre Wildtyp- und Bibliothek-AAV-Kapside (AAV-Transfer-Shuttle) verpackt, indem 2x10⁸ 293T-Zellen in 10 Zellkulturschalen (15 cm) mit dem Plasmid pVP3cm (enthält das Wildtyp-Cap-Gen mit modifizierter Kodonnutzung ohne die terminalen inverted repeats) [27], dem Bibliothek-Plasmiden und dem pXX6-Helferplasmid [28] transfiziert wurden, wobei das Verhältnis zwischen den Plasmiden 1:1:2 betrug. Die resultierenden AAV-Bibliothek-Transfer-Shuttle wurden dazu verwendet, 2x10⁸ 293T-Zellen in Zellkulturschalen (15 cm) bei einer MOI von 0,5 replikativen Einheiten pro Zelle zu infizieren. Die Zellen wurden mit Ad5 (bereitgestellt durch das Laboratoire des Therapie Genique, Frankreich) bei einer MOI von 5 Plaque-bildenden Einheiten (pfu/Zelle) superinfiziert. Die finale AAV-Display-Bibliothek wurde nach 48 Stunden aus den Überständen geerntet. Die Überstände wurden unter Verwendung von VivaSpin-Säulen (Viva Science, Hannover, Deutschland) aufkonzentriert und durch Iodixanol-Dichtegradienten-Ultrazentrifugation wie zuvor beschrieben [29] aufgereinigt und durch real-time-PCR unter Verwendung der cap-spezifischen Primer 5' - GCAGTATGGTTCTGTATCTACCAACC - 3' (SEQ ID NO:13) und 5' - GCCTGGAAGAACGCCTTGTGTG - 3' (SEQ ID NO:14) mit dem LightCycler-System (Roche Diagnostics, Mannheim, Deutschland) titriert.

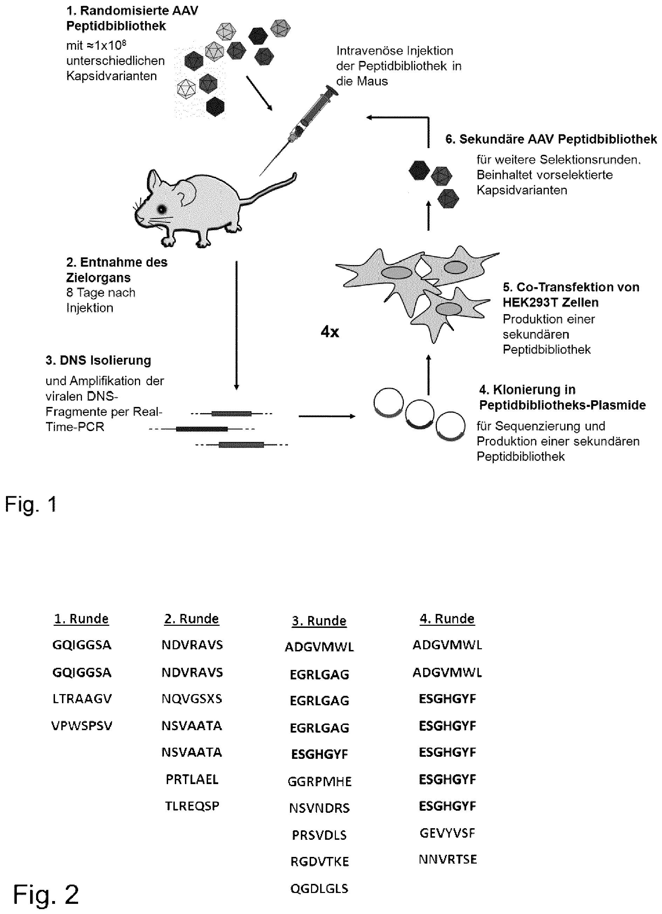

Für das Biopanning *in vivo* wurden 1x10¹¹ Partikel der genomischen Bibliothek in die Schwanzvene von FVB/N-Mäusen injiziert. Den Partikeln wurde 8 Tage Zeit für die Verteilung und die Infektion der Zielzellen gegeben. Nach 8 Tagen wurden die Mäuse getötet und die Lungen wurden entfernt. Die Gesamt-DNA des Gewebes wurde unter Verwendung des DNeasy Tissue Kits (Qiagen) extrahiert. Die Zufallsoligonukleotide, die in AAV-Partikeln der Bibliothek enthalten waren und sich in dem Gewebe von Interesse angereichert hatten, wurden durch Nested-PCR unter Verwendung der Primer 5'-ATGGCAAGCCACAAGGACGATG-3' (SEQ ID NO:15) und 5' - CGTGGAGTACTGTGTGATGAAG - 3' (SEQ ID NO:16) für die erste PCR und den Primern 5' - GGTTCTCATCTTTGGGAAGCAAG - 3' (SEQ ID NO:17) und 5'-TGATGAGAATCTGTGGAGGAG - 3' (SEQ ID NO:18) für die zweite PCR amplifiziert. Die mittels PCR amplifizierten Oligonukleotide wurden verwendet, um sekundäre Bibliothek für drei weitere Selektionsrunden herzustellen. Die sekundären Bibliotheken wurden wie die primären Bibliotheken erzeugt (siehe oben), jedoch ohne den zusätzlichen Schritt der Herstellung von Transfer-Shutteln. Die sekundäre Plasmid-Bibliothek wurde verwendet, um 2x10⁸ 293T-Zellen in Zellkulturschalen (15 cm) bei einem Verhältnis von 25 Bibliothek-Plasmiden pro Zelle zu transfizieren, wobei das Transfektionsmittel Polyfect (Qiagen) verwendet wurde. Nach jeder Selektionsrunde wurden einige Klone sequenziert. Das angewendete Selektionsverfahren ist in Fig. 1 abgebildet.

Ergebnisse: Nach vier Selektionsrunden wurden insgesamt 9 Klone sequenziert. Die Sequenzierung ergab, dass 5 Klone die Peptidsequenz ESGHGYF (SEQ ID NO:2) aufwiesen. Weitere Klone zeigten die Peptidsequenzen ADGVMWL (SEQ ID NO:3), GEVYVSF (SEQ ID NO:4) und NNVRTSE (SEQ ID NO:5). Drei der vier Peptidsequenzen, einschließlich des dominanten Klons ESGHGYF, sowie auch ADGVMWL und GEVYVSF zeigten mindestens einen hydrophoben aromatischen Rest. Die in den verschiedenen Selektionsrunden gewonnenen Peptide sind in Fig. 2 gezeigt.

### Beispiel 2: Herstellung und Quantifizierung rekombinanter AAV-Vektoren

Die in Beispiel 1 angereicherten Klone wurden als rekombinante AAV-Vektoren hergestellt und auf ihr Transduktionsprofil untersucht. Rekombinante AAV-Vektoren wurden durch dreifache Transfektion von HEK293T-Zellen erzeugt. Die Zellen wurden bei 37°C, 5% CO₂ in Dulbeccos modifiziertem Eagle Medium (Invitrogen, Carlsbad, USA), das mit 1% Penicillin/Streptomycin und 10% fetalem Kälberserum supplementiert war, angezüchtet. Plasmid-DNA wurde mit dem Transfektionsmittel Polyfect (Qiagen, Hilden, Deutschland) in 293T-Zellen transfiziert. Vier Tage nach der Transfektion wurden die Zellen geerntet, lysiert und die Vektoren wurden mittels Iodixanol-Dichtegradienten-Ultrazentrifugation aufgereinigt, wie es zuvor beschrieben wurde [29]. Für die Transfektionen wurde pXX6 als adenovirales Helfer-Plasmid [28], das Luciferase-Gen pUF2-CMV-luc [27] oder das GFP-Gen pTR-CMV-GFP [30], sowie ein Plasmid, das für das AAV-Kapsid von Interesse kodiert, verwendet. Plasmide, die für die AAV-Kapsid-Mutanten kodierten, welche zuvor aus der AAV-Bibliothek selektiert worden waren, und Wildtyp-Kontrollen waren modifiziertes pXX2-187 [31] bzw. pXX2 [28]. Für ein Alanin-Scanning wurden ferner Oligonukleotid-Inserts hergestellt, die für modifizierte Varianten des Peptids ESGHGYF kodieren. Die Inserts wurden wie beschrieben zu Bibliothek-Inserts verarbeitet (siehe oben). Für die Quantifizierung der rekombinanten Vektoren wurden die genomischen Titer durch quantitative real-time PCR unter Verwendung der CMV-spezifischen Primer 5' - GGCGGAGTTGTTACGACAT - 3' (SEQ ID NO:19) und 5' - GGGACTTTCCCTACTTGGCA - 3' (SEQ ID NO:20) mit dem LightCycler-System bestimmt, wie es zuvor beschrieben wurde [32].

### Beispiel 3: Untersuchung des Tropismus der rekombinanten AAV-Vektoren in vivo

Um den Tropismus der angereicherten Peptide *in vivo* untersuchen zu können, wurden die Peptide in das Kapsid eines rekombinanten Vektors eingebracht, der ein Luciferase-Reporter-Gen umfasst. Vektoren mit mutierten Kapsiden sowie Kontrollvektoren wurden in Mäuse injiziert. Die AAV-Vektoren wurden intravenös in einer Dosis von 5x10¹⁰ Vektorgenomen (vg)/Maus verabreicht (n=3 Tiere pro injiziertem AAV-Klon). Am Tag 14 wurden die Tiere mit Isofluran betäubt. Die Luciferase-Expression wurde unter Verwendung eines Xenogen IVIS200-Imaging Systems (Caliper Lifescience, Hopkinton, USA) mit der Software Living Image 4.0 (Caliper) nach intraperitonialer Injektion von 200 µl Luciferin-Substrat (150 mg/kg, Xenogen) pro Maus analysiert. Es wurden repräsentative *in vivo* Biolumineszenz-Abbildungen der Expression des Transgens an unterschiedlichen Positionen (ventral, dorsal, lateral) aufgenommen, wenn die Lumineszenz in relativen Lichteinheiten (Photonen/Sek/cm²) die höchste Intensität erreichte. Anschließend wurden die Tiere getötet, die Organe von Interesse wurden schnell entfernt, und Abbildungen der Expression des Transgens in einzelnen Organen wurden sofort aufgenommen. Anschließend wurden die Organe in flüssigem Stickstoff eingefroren und bei -80°C gelagert. Dreidimensionale Rekonstruktionen der *in vivo* Lumineszenz-Abbildungen wurden erhalten, indem man die DLIT-Option der Software Living Image 4.0 verwendete und das emittierte Licht in 5 verschiedenen Wellenlängen von 560-640 nm für jeweils drei Minuten gemessen wurde. Um die Luciferase-Expression zu quantifizieren wurden die Organe in Reporter-Lysepuffer (RLB, Promega, Madison, USA) homogenisiert. Die Bestimmung der Luciferase-Reporter-Gen-Aktivität wurde in ein Luminometer (Mithras LB9 40, Berthold Technologies, Bad Wildbad, Deutschland) in 10-Sekunden Intervallen nach Zugabe von 100 µm Luciferase-Assay-Reagenz (LAR, Promega) durchgeführt, wobei zwischen den Messungen jeweils 2 Sekunden Verzögerung bestand. Die Werte wurden in Bezug auf die Gesamtproteinmenge in jeder Probe normalisiert, wobei der Roti Nanoquant-Proteinassay (Roth, Karlsruhe, Deutschland) verwendet wurde.

Ergebnisse: Es wurde herausgefunden, dass die Ausbeute in Bezug auf die Vektor-Titer bei rekombinanten Vektoren mit Luciferase-Reporter-Gen vergleichbar zu Vektoren war, die ein Wildtyp-AAV2-Kapsid trugen, was darauf schließen ließ, dass die angereicherten Peptide den Zusammenbau des Kapsids oder die Verpackung des Gens nicht negativ beeinflussen. Die *in vivo* Messung der Biolumineszenz nach 14 Tagen ergab, dass das Peptid ESGHGYF für eine starke und lungenspezifische Expression des Transgens sorgte (≤10⁵p/sek/cm²/r). Diese Ergebnisse wurden durch die *ex vivo* durchgeführten Kontrollversuche mit explantierten Organen bestätigt. Ein auf Zufallsbasis ausgewählter Kontrollklon der nicht-selektierten Bibliothek (CVGSPCG) führte zu einer schwachen Gen-Expression, die vornehmlich im Herz und an einigen Stellen des Abdomens auftrat, nicht jedoch in der Lunge. Wildtyp-AAV2 verursachte eine schwache Gen-Expression in Herz, Leber und Skelettmuskel, nicht jedoch in der Lunge. Eine dreidimensionale Rekonstruktion der Biolumineszenz-Abbildungen bestätigte die lungenspezifische Expression. Das Peptid ADGVMWL (SEQ ID NO:3), das ebenfalls während der in vivo Selektion angereichert wurde, führte auch zu einer lungenspezifischen. Expression des Transgens, die jedoch schwächer war als beim Peptid ESGHGYF. Während die Gen-Expression 14 Tage nach Verabreichung des ADGVMWL-Luciferase-Vektors sehr gering war, stieg sie auf etwa 5x10⁴ p/sek/cm²/r an und konnte 28 Tage nach Vektorinjektion spezifisch in der Lunge beobachtet werden. Diese Ergebnisse wurden durch die *ex vivo* durchgeführten Kontrollversuche mit explantierten Organen bestätigt. Die Untersuchung der Luciferase-Aktivität von Gewebelysaten aus repräsentativen Organen ergab, dass Wildtyp-AAV2 im Herz eine geringe Gen-Expression verursachte (2,9x10⁴ RLU/mg Protein, siehe Fig. 3A, obere Tafel) und noch geringere Expressionswerte in anderen Organen. Das Kontrollpeptid CVSGPCG vermittelte eine moderate Gen-Expression im Herz (8,7x10⁴ RLU/mg Protein, siehe Fig. 3A, mittlere Tafel). Im Gegensatz dazu führten Vektoren, die das lungenspezifische ESGHGYF-Kapsid aufwiesen, zu einer starken und spezifischen Gen-Expression in der Lunge (4,1x10⁵ RLU/mg Protein, siehe Fig. 3A, untere Tafel). Im Herz und in der Leber (d.h. in den beiden Organen, in denen Wildtyp-AAV2 und der Peptidvektor CVGSPCG zu einer starken Expression führen) zeigten die lungenspezifischen ESGHGYF-Vektoren lediglich eine Expression im Bereich des Hintergrundsignals (etwa 1x10³ RLU/mg Protein). Im Gegensatz dazu war die Expression des Transgens in der Lunge bei den ESGHGYF-Vektoren mehr als 200-fach höher als die Expression, die durch Wildtyp-AAV2 oder durch die die CVGSPCG-Kontrollvektoren vermittelt wurden (siehe Fig. 3B).

Die Ergebnisse zeigten ferner, dass die durch die den ESGHGYF-Vektoren vermittelte lungenspezifische Expression des Transgens auch über einen langen Zeitraum organspezifisch blieb. Nach intravenöser Verabreichung der lungenspezifischen AAV2-ESGHGYF-Luciferasevektoren wurde die Expression des Transgens über einen Zeitraum von 164 Tagen gemessen. Die im Bereich der Lunge emittierte Strahlung wurde dabei quantitativ erfasst. Über den gesamten Zeitraum hinweg erfolgte die Expression des Transgens stabil auf hohem Niveau, und sie war auf die Lunge beschränkt. Die geringste Expression in der Lunge wurde am Tag 7 gemessen, ein Peak wurde am Tag 42 erreicht, und die Strahlung nahm bis zur letzten Messung am Tag 164 nur langsam ab (Fig. 4).

### Beispiel 4: Alanin-Scanning beim Peptid ESGHGYF

Um die Bedeutung der einzelnen Aminosäuren in dem Peptid ESGHGYF in Bezug auf die Lungenspezifität zu untersuchen, wurde ein Alanin-Scanning durchgeführt.

Ergebnisse: Es wurde festgestellt, dass durch Austausch der ersten beiden Aminosäuren der Lungen-spezifische Tropismus nicht verändert wurde. Wurden hingegen die Aminosäuren 3-4 oder 5-7 ausgetauscht, so kam es entweder zu einem Totalverlust der Infektionsfähigkeit (Position 3 oder 4) oder zu einer Veränderung der Spezifität zu Herz oder Skelettmuskel (Positionen 5-7) .

### Beispiel 5: Analyse der Vektor-Verteilung

Um nachzuprüfen, ob die Lungenspezifische Expression des Transgens von intravenös injizierten ESGHGYF-Vektoren auf einem lungenspezifischen Homing basiert, wurde zunächst die Verteilung der Vektoren vier Stunden nach intravenöser Verabreichung von 5x10¹⁰ gp/Maus untersucht. Die Quantifizierung der Vektorgenome erfolgte durch real-time PCR. Zunächst wurde zu verschiedenen Zeitpunkten nach intravenöser Verabreichung von 5x10¹⁰ vg/Maus die Gesamt-DNA aus dem betreffenden Organ unter Verwendung eines Gewebehomogenisators (Precellys 24, Peqlab, Erlangen, Deutschland) und des DNeasy Tissue Kits (Qiagen, Hilden, Deutschland) gemäß den Anweisungen des Herstellers extrahiert. Die DNA wurde unter Verwendung eines Spektralphotometers (Nanodrop ND-2000C, Peqlab) quantifiziert. Die Analyse der AAV-Vektor-DNA in den Geweben wurde durch quantitative real-time PCR unter Verwendung der zuvor beschriebenen CMV-spezifischen Primer durchgeführt, wobei 40 ng Template verwendet wurden, die in Bezug auf die Gesamt-DNA normalisiert wurden.

Ergebnisse: Die Quantifizierung der Vektorgenome durch real-time PCR zeigte ein lungenspezifisches Homing von ESGHGYF. Die Menge an Vektorgenomen, die in der Lunge nachgewiesen werden konnten (3,8x10⁵ ± 1,9x10⁵ vg/100ng Gesamt-DNA) war etwa 6-100fach höher als die Menge an Vektorgenomen, die in einem anderen Organ nachgewiesen wurde (Fig. 5A). Um die direkte Korrelation zwischen Vektor-Homing und Expression des Transgens zu ermitteln, wurde die Vektorverteilung von Wildtyp-AAV2, des Kontrollpeptids CVGSPCG und des lungenspezifischen Peptids ESGHGYF 14 Tage nach intravenöser Verabreichung von 5x 0¹⁰ gp/Maus gemessen, d.h. zu dem Zeitpunkt, an dem die Expression des Transgens bestimmt wurde (siehe oben). Die von Wildtyp-AAV2-Vektoren bereitgestellten Genome konnten hauptsächlich aus Leber und Milz gewonnen werden, wobei auch die Genome von Vektoren, die das Kontrollpeptid aufwiesen, hauptsächlich aus der Milz gewonnen wurden. Insgesamt waren die Menge an VektorGenomen, die in der Milz nachgewiesen wurden, bei allen untersuchten Kapsid-Varianten verhältnismäßig gleich (4x10³ vp/100 ng Gesamt-DNA), was auf einen unspezifischen Aufnahmemechanismus für die Partikel im retikulo-endothelialen System verweist, der unabhängig von der Bereitstellung und der Expression des Transgens ist. Im Gegensatz dazu ähneln die Verteilungsdaten von Genomen, die durch Vektoren bereitgestellt wurden, welche das lungenspezifische Peptid ESGHGYF aufwiesen, stark den Expressionsdaten des Transgens, wobei eine hochspezifische Anreicherung in der Lunge beobachtet werden kann. Die Menge von in der Lunge nachgewiesenen Vektoren, die das Peptid ESGHGYF zeigten, war etwa 250-fach höher als in anderen Organen und bis zu 500-fach höher als in Lungen, denen ein Wildtyp-Vektor oder ein Kontroll-Kapsid-Vektor injiziert wurde (Fig. 5B). Dieselben Verteilungswerte zwischen den Organen wurden 28 Tage nach Vektorverabreichung nachgewiesen. Der direkte Vergleich der Mengen der nachgewiesenen Genome zwischen den drei Vektor-Kapsid-Varianten ist in Fig. 5C für die drei Gewebe gezeigt, in denen relevante Mengen Vektor-DNA detektiert werden konnte. Insgesamt zeigen diese Daten, dass eine lungenspezifische Expression des Transgens, die durch ESGHGYF-Vektoren vermittelt wird, durch ein Gewebe-spezifisches Homing von zirkulierenden Partikeln erreicht wird.

### Beispiel 6: Immunhistochemie und Histologie

Es wurde eine Immunhistochemie durchgeführt, um 14 Tage nach intravenöser Verabreichung des rAAV-GFP-Vektors, der das Peptid ESGHGYF aufweist, bzw. des Wildtyp-AAV-Kapsids als Kontrolle die Expression des Transgens auf zellulärer Ebene in der Lunge sowie in einem Kontroll-Organ sichtbar zu machen. Die Lungen der Tiere wurden ex situ mit 4% (w/v) Paraformaldehyd über die Luftröhre unter hydrostatischem Druck einer 20 cm Wassersäule für 20 Minuten fixiert, gefolgt von 24 Stunden Immersion im selben Fixierungsmittel. Die Lungengewebe wurden in Paraffin eingebettet. Schnitte einer Stärke von 2 µm wurden von Wachs entfernt, rehydratisiert und für die Immunhistochemie verwendet. Die Immunhistochemie wurde mit polyklonalen Antikörpern gegen GFP (A-11122, Invitrogen) oder CD31 (AB28364, Abcam, Cambridge, USA) durchgeführt. Die Aktivität der endogenen Peroxidase wurde mit 1% H₂O₂ in Methanol für 30 Minuten inaktiviert. Vor der Färbung mit CD31 wurden die Schnitte in Citratpuffer (pH 6,0) für 20 Minuten auf 100°C erhitzt. Nach Waschen in PBS wurden die Schnitte für 30 Minuten mit PBS, 10% Ziegenserum (Vector Lab, Burlingame, USA) und 2% Milchpulver (Roth) inkubiert. Primären Antikörpern wurde es erlaubt, für 1 Stunde bei 37°C zu binden. Nach Waschen in PBS wurden die Schnitte für 30 Minuten mit einem sekundären, biotinylierten Ziege-Anti-Kaninchen-Antikörper (Vector Lab) inkubiert. Gebundene Antikörper wurden unter Verwendung des VECTASTAIN-Elite-ABC-Kits (Vector Lab) und 3,3'-Diaminobenzidin (DAB, Sigma-Aldrich, St. Louis, USA) visualisiert. Ausgewählte Schnitte wurden mit Hemalum gegengefärbt.

Ergebnisse: In Lungen von Mäusen, denen rAAV-ESGHGYF injiziert wurde, ergab eine mikroskopische Untersuchung eine intensive Färbung der Endothelzellen über die gesamte pulmonale Mikrovaskulatur hinweg und in einem geringfügig geringeren Ausmaß in den großen pulmonalen Gefäßen (Daten nicht gezeigt). Im Gegensatz dazu zeigte pulmonales Gewebe von Mäusen, denen Wildtyp-AAV2-Vektor injiziert wurde, keine Färbung. Um die Gewebespezifität zu bestätigen, wurde die Leber als Kontrollorgan analysiert (ein Gewebe, von dem bekannt ist, dass es häufig eine hohe Expression eines Transgens nach Injektion von Wildtyp-AAV2-Vektor zeigt. In der Leber wurde eine Färbung von Hepatozyten nach der Verabreichung von Wildtyp-RAAV2-Vektor beobachtet, jedoch keine Färbung nach Verabreichung des rAAV2-ESGHGYF-Vektors. Die endotheliale Abstammung der mit den Vektoren transduzierten pulmonalen Zellen wurde durch CD31-Färbung bestätigt, wobei das durch die GFP-Färbung erhaltene Muster in seriellen Schnitten der Lunge von Mäusen, denen rAAV2-ESGHGYF injiziert wurde, bestätigt wurde (Daten nicht gezeigt).

### LITERATURLISTE

[1] Barst et al., 2004, J Am Coll Cardiol, 43: 40-47
[2] McLaughlin et al., 2009, Circulation, 119: 2250-2294
[3] Stenmark et al., 2009, Am J Physiol Lung Cell Mol Physiol, 297: 1013-1032
[4] Friedman et al., 2012, J Obes, 2012: 505274
[5] Chin et al., 2005, Coron Artery Dis, 16: 13-18.
[6] Hemnes et al., 2008, Int J Clin Pract Suppl: 11-19
[7] Humbert et al., 2009, Am J Respir Crit Care Med, 179: 650-656
[8] Simonneau et al., 2009, J Am Coll Cardiol, 54: 43-54
[9] Humbert et al., 2006, Am J Respir Crit Care Med, 173: 1023-1030
[10] Tenenbaum et al., 2003, Curr Gene Ther, 3:545-565
[11] Work et al., 2006, Mol Ther, 4:683-693
[12] Shi et al., 2006, Hum Gene Ther, 17:353-361
[13] US 2007/0172460 A1
[14] Michelfelder et al., 2009, PLOS One, 4(4):e5122
[15] Shi and Bartlett, 2003, Mol Ther, 7:515-525
[16] Loiler et al., 2003, Gene Ther, 10:1551-1558
[17] Rabinowitz et al., 1999, Virology, 265:274-285
[18] Wu et al., 2000, J Virol, 74:8635-8647
[19] Shi et al., 2001, Hum Gene Ther, 12:1697-1711
[20] Warrington et al., 2004, J Virol, 78:6595-6609
[21] Girod et al., 1999, Nat Med, 5:1052-1056
[22] Grifman et al., 2001, Mol Ther, 3:964-975
[23] Opie et al., 2003, J Virol, 77:6995-7006
[24] Kern et al., 2003, J Virol, 77:11072-11081
[25] Russell et al., 1998, J Virol, 72:309-319)
[26] Müller et al., 2003, Nat Biotechnol 21, 1040 - 1046
[27] Waterkamp, et al., 2006, J Gene Med 8, 1307-1319
[28] Xiao et al., 1998, Journal of Virology 72, 2224-2232
[29] Zolotukhin, et al., 1999, Gene Ther 6, 973 - 985
[30] McCarty et al., 2001, Gene Ther 8, 1248-1254
[31] Michelfelder, et al., 2007, Exp Hematol 35, 1766 - 1776
[32] Rohr et al., 2005, J Virol Methods 127, 40 - 45

Ausführungsformen der vorliegenden Erfindung sind:
1. Peptid, Polypeptid oder Protein, das spezifisch an Zellen der Lunge bindet, dadurch gekennzeichnet, dass es die Aminosäuresequenz von SEQ ID NO:1 umfasst.
2. Peptid, Polypeptid oder Protein nach Ausführungsform 1, das die Aminosäuresequenz von SEQ ID NO:2 umfasst oder eine Variante davon, die sich von der Aminosäuresequenz von SEQ ID NO:2 durch Modifikation von mindestens einer der beiden N-terminalen Aminosäure unterscheidet.
3. Protein, das nach Ausführungsform 1 oder 2, wobei es sich um ein Kapsid-Protein eines viralen Vektors handelt.
4. Protein nach Ausführungsform 3, wobei es sich um ein Kapsid-Protein eines Adeno-assoziierten Virus (AAV) handelt.
5. Protein nach Ausführungsform 4, wobei es sich um ein Kapsid-Protein eines AAV von einem Serotyp ausgewählt aus der Gruppe bestehend aus den 2, 4, 6, 8 und 9 handelt.
6. Protein nach Ausführungsform 5, wobei es sich um ein Kapsid-Protein eines AAV vom Serotyp 2 handelt.
7. Protein nach Ausführungsform 6, wobei es sich um das VP1-Protein eines AAV vom Serotyp 2 handelt.
8. Protein nach einer der Ausführungsformen 1-7, wobei das Peptid im Bereich der Aminosäuren 550-600 des Kapsids vorliegt.
9. Protein nach einer der Ausführungsformen 1-8, das folgendes umfasst:
   (a) die Aminosäuresequenz von SEQ ID NO:9;
   (b) eine Aminosäuresequenz, die mindestens 80% Identität zu der Aminosäuresequenz von SEQ ID NO:9 aufweist; oder
   (c) ein Fragment von einer der in (a) oder (b) definierten Aminosäuresequenzen.
10. Virales Kapsid, das ein Peptid, Polypeptid oder Protein nach einer der Ausführungsformen 1-9 umfasst.
11. Nukleinsäure, die für ein Peptid, Polypeptid oder Protein nach einer der Ausführungsformen 1-9 kodiert.
12. Plasmid, das eine Nukleinsäure nach Ausführungsform 11 umfasst.
13. Rekombinanter viraler Vektor, der ein Kapsid und ein darin verpacktes Transgen umfasst, wobei das Kapsid mindestens ein Kapsid-Protein umfasst, welches die Aminosäuresequenz von SEQ ID NO:1 oder SEQ ID NO:2 oder eine Variante davon umfasst, die sich von der Aminosäuresequenz von SEQ ID NO:2 durch Modifikation von mindestens einer der beiden N-terminalen Aminosäure unterscheidet.
14. Rekombinanter viraler Vektor nach Ausführungsform 13, wobei es sich um einen rekombinanten AAV-Vektor handelt.
15. Rekombinanter AAV-Vektor nach Ausführungsform 14, wobei es sich um einen AAV-Vektor eines Serotyps ausgewählt aus der Gruppe bestehend aus den Serotypen 2, 4, 6, 8 und 9 handelt.
16. Rekombinanter AAV-Vektor nach Ausführungsform 15, wobei es sich um einen AAV-Vektor vom Serotyp 2 handelt.
17. Rekombinanter AAV-Vektor nach einer der Ausführungsformen 13-16, wobei das Transgen für eine Stickstoffmonoxidsynthase oder für den Bone-Morphogenic-Protein-Rezeptor 2 (BMPR2) kodiert.
18. Rekombinanter AAV-Vektor nach Ausführungsform 17, wobei das Transgen für die endotheliale Stickstoffmonoxidsynthase (eNOS) oder die induzierbare Stickstoffmonoxidsynthase (iNOS) kodiert.
19. Rekombinanter AAV-Vektor nach einer der Ausführungsformen 13-18, wobei das Transgen in Form einer ssDNA oder einer dsDNA vorliegt.
20. Rekombinanter AAV-Vektor nach einer der Ausführungsformen 13-19 zur Verwendung in einem Verfahren zur Behandlung einer Lungenfunktionsstörung oder einer Lungenerkrankung in einem Subjekt.
21. Rekombinanter AAV-Vektor zur Verwendung in einem Verfahren nach Ausführungsform 20, wobei die Lungenerkrankung eine pulmonale Hypertonie oder eine pulmonal-arterielle Hypertonie ist.
22. Rekombinanter AAV-Vektor zur Verwendung in einem Verfahren nach einer der Ausführungsformen 20-21, wobei das Subjekt ein Säugetier, vorzugsweise ein Mensch ist.
23. Rekombinanter AAV-Vektor zur Verwendung in einem Verfahren nach einer der Ausführungsformen 20-22, wobei der Vektor für die intravenöse Verabreichung formuliert ist.
24. Zelle, die ein Peptid, Polypeptid oder Protein nach einer der Ausführungsformen 1-9, ein virales Kapsid nach Ausführungsform 10, eine Nukleotidsequenz nach Ausführungsform 11, ein Plasmid nach Ausführungsform 12, oder einen rekombinanten AAV-Vektor nach einer der Ausführungsformen 13-19 umfasst.
25. Pharmazeutische Zusammensetzung, die ein Peptid, Polypeptid oder Protein nach einer der Ausführungsformen 1-9, ein virales Kapsid nach Ausführungsform 10, eine Nukleotidsequenz nach Ausführungsform 11, ein Plasmid nach Ausführungsform 12, oder einen rekombinanten AAV-Vektor nach einer der Ausführungsformen 13-19 umfasst.
26. Verwendung eines Peptids, Polypeptids oder Proteins nach einer der Ausführungsformen 1-9, eines viralen Kapsids nach Ausführungsform 10, einer Nukleotidsequenz nach Ausführungsform 11, eines Plasmids nach Ausführungsform 12 oder eines rekombinanten AAV-Vektors nach einer der Ausführungsformen 13-19 zur Behandlung einer Lungenfunktionsstörung oder einer Lungenerkrankung in einem Subjekt.
27. Verwendung nach Ausführungsform 26, wobei die Lungenerkrankung eine pulmonale Hypertonie oder eine pulmonal-arterielle Hypertonie ist.

## Patentansprüche

1. Peptid, Polypeptid oder Protein, das spezifisch an Zellen der Lunge bindet, **dadurch gekennzeichnet, dass** es die Aminosäuresequenz von SEQ ID NO:3, 4 oder 5 umfasst.

2. Protein, das nach Anspruch 1, wobei es sich um ein Kapsid-Protein eines viralen Vektors handelt.

3. Protein nach Anspruch 2, wobei es sich um ein Kapsid-Protein eines Adeno-assoziierten Virus (AAV) handelt.

4. Protein nach Anspruch 3, wobei es sich um ein Kapsid-Protein eines AAV von einem Serotyp ausgewählt aus der Gruppe bestehend aus den 2, 4, 6, 8 und 9 handelt.

5. Virales Kapsid, das ein Peptid, Polypeptid oder Protein nach einem der Ansprüche 1-4 umfasst.

6. Nukleinsäure, die für ein Peptid, Polypeptid oder Protein nach einem der Ansprüche 1-4 kodiert.

7. Plasmid, das eine Nukleinsäure nach Anspruch 6 umfasst.

8. Rekombinanter viraler Vektor, der ein Kapsid und ein darin verpacktes Transgen umfasst, wobei das Kapsid mindestens ein Kapsid-Protein umfasst, welches die Aminosäuresequenz von SEQ ID NO:3, 4 oder 5 umfasst.

9. Rekombinanter viraler Vektor nach Anspruch 8, wobei es sich um einen rekombinanten AAV-Vektor handelt.

10. Rekombinanter AAV-Vektor nach Anspruch 9, wobei es sich um einen AAV-Vektor eines Serotyps ausgewählt aus der Gruppe bestehend aus den Serotypen 2, 4, 6, 8 und 9 handelt.

11. Rekombinanter AAV-Vektor nach einem der Ansprüche 8-10 zur Verwendung in einem Verfahren zur Behandlung einer Lungenfunktionsstörung oder einer Lungenerkrankung in einem Subjekt.

12. Zelle, die ein Peptid, Polypeptid oder Protein nach einem der Ansprüche 1-4, ein virales Kapsid nach Anspruch 5, eine Nukleotidsequenz nach Anspruch 6, ein Plasmid nach Anspruch 7, oder einen rekombinanten AAV-Vektor nach einem der Ansprüche 8-10 umfasst.

13. Pharmazeutische Zusammensetzung, die ein Peptid, Polypeptid oder Protein nach einem der Ansprüche 1-4, ein virales Kapsid nach Anspruch 5, eine Nukleotidsequenz nach Anspruch 6, ein Plasmid nach Anspruch 7, oder einen rekombinanten AAV-Vektor nach einem der Ansprüche 8-10 umfasst.

14. Peptid, Polypeptid oder Protein nach einem der Ansprüche 1-4, virales Kapsid nach Anspruch 5, Nukleotidsequenz nach Anspruch 6, Plasmid nach Anspruch 7 oder rekombinanter AAV-Vektor nach einem der Ansprüche 8-10 zur Verwendung in einem Verfahren der Behandlung einer Lungenfunktionsstörung oder einer Lungenerkrankung in einem Subjekt.

15. Peptid, Polypeptid oder Protein nach einem der Ansprüche 1-4, virales Kapsid nach Anspruch 5, Nukleotidsequenz nach Anspruch 6, Plasmid nach Anspruch 7 oder rekombinanter AAV-Vektor nach einem der Ansprüche 8-10 zur Verwendung in einem Verfahren nach Anspruch 14, wobei die Lungenerkrankung eine pulmonale Hypertonie oder eine pulmonal-arterielle Hypertonie ist.


SEQUENCE LISTING

<110>  Boehringer Ingelheim International GmbH

<120>  Neue Peptide mit Spezifität für die Lunge

<130>  P 106929

<160>  20

<170>  PatentIn version 3.5

<210>  1
<211>  5
<212>  PRT
<213>  Artificial Sequence

<220>
<223>  Lungenspezifisches Peptid

<400>  1

Gly His Gly Tyr Phe
1               5

<210>  2
<211>  7
<212>  PRT
<213>  Artificial Sequence

<220>
<223>  Lungenspezifisches Peptid

<400>  2

Glu Ser Gly His Gly Tyr Phe
1               5

<210>  3
<211>  7
<212>  PRT
<213>  Artificial Sequence

<220>
<223>  Lungenspezifisches Peptid

<400>  3

Ala Asp Gly Val Met Trp Leu
1               5

<210>  4
<211>  7
<212>  PRT
<213>  Artificial Sequence

<220>
<223>  Lungenspezifisches Peptid

<400>  4

Gly Glu Val Tyr Val Ser Phe
1               5

<210>  5
<211>  7
<212>  PRT
<213>  Artificial Sequence

<220>
<223>  Lungenspezifisches Peptid

<400>  5

Asn Asn Val Arg Thr Ser Glu
1               5

<210>  6
<211>  735
<212>  PRT
<213>  adeno-associated virus 2

<400>  6

<210> 7
<211> 598
<212> PRT
<213> adeno-associated virus 2

<400> 7

Met Ala Pro Gly Lys Lys Arg Pro Val Glu His Ser Pro Val Glu Pro
1               5                   10                  15

Asp Ser Ser Ser Gly Thr Gly Lys Ala Gly Gln Gln Pro Ala Arg Lys
            20                  25                  30

Arg Leu Asn Phe Gly Gln Thr Gly Asp Ala Asp Ser Val Pro Asp Pro
        35                  40                  45

Gln Pro Leu Gly Gln Pro Pro Ala Ala Pro Ser Gly Leu Gly Thr Asn
    50                  55                  60

Thr Met Ala Thr Gly Ser Gly Ala Pro Met Ala Asp Asn Asn Glu Gly
65                  70                  75                  80

Ala Asp Gly Val Gly Asn Ser Ser Gly Asn Trp His Cys Asp Ser Thr
                85                  90                  95

Trp Met Gly Asp Arg Val Ile Thr Thr Ser Thr Arg Thr Trp Ala Leu
            100                 105                 110

Pro Thr Tyr Asn Asn His Leu Tyr Lys Gln Ile Ser Ser Gln Ser Gly
        115                 120                 125

Ala Ser Asn Asp Asn His Tyr Phe Gly Tyr Ser Thr Pro Trp Gly Tyr
    130                 135                 140

Phe Asp Phe Asn Arg Phe His Cys His Phe Ser Pro Arg Asp Trp Gln
145                 150                 155                 160

Arg Leu Ile Asn Asn Asn Trp Gly Phe Arg Pro Lys Arg Leu Asn Phe
                165                 170                 175

Lys Leu Phe Asn Ile Gln Val Lys Glu Val Thr Gln Asn Asp Gly Thr
            180                 185                 190

Thr Thr Ile Ala Asn Asn Leu Thr Ser Thr Val Gln Val Phe Thr Asp
        195                 200                 205

Ser Glu Tyr Gln Leu Pro Tyr Val Leu Gly Ser Ala His Gln Gly Cys
    210                 215                 220

Leu Pro Pro Phe Pro Ala Asp Val Phe Met Val Pro Gln Tyr Gly Tyr
225                 230                 235                 240

Leu Thr Leu Asn Asn Gly Ser Gln Ala Val Gly Arg Ser Ser Phe Tyr
                245                 250                 255

Cys Leu Glu Tyr Phe Pro Ser Gln Met Leu Arg Thr Gly Asn Asn Phe
            260                 265                 270

Thr Phe Ser Tyr Thr Phe Glu Asp Val Pro Phe His Ser Ser Tyr Ala
        275                 280                 285

His Ser Gln Ser Leu Asp Arg Leu Met Asn Pro Leu Ile Asp Gln Tyr
    290                 295                 300

Leu Tyr Tyr Leu Ser Arg Thr Asn Thr Pro Ser Gly Thr Thr Thr Gln
305                 310                 315                 320

Ser Arg Leu Gln Phe Ser Gln Ala Gly Ala Ser Asp Ile Arg Asp Gln
                325                 330                 335

Ser Arg Asn Trp Leu Pro Gly Pro Cys Tyr Arg Gln Gln Arg Val Ser
            340                 345                 350

Lys Thr Ser Ala Asp Asn Asn Asn Ser Glu Tyr Ser Trp Thr Gly Ala
        355                 360                 365

Thr Lys Tyr His Leu Asn Gly Arg Asp Ser Leu Val Asn Pro Gly Pro
    370                 375                 380

Ala Met Ala Ser His Lys Asp Asp Glu Glu Lys Phe Phe Pro Gln Ser
385                 390                 395                 400

Gly Val Leu Ile Phe Gly Lys Gln Gly Ser Glu Lys Thr Asn Val Asp
                405                 410                 415

Ile Glu Lys Val Met Ile Thr Asp Glu Glu Glu Ile Arg Thr Thr Asn
            420                 425                 430

Pro Val Ala Thr Glu Gln Tyr Gly Ser Val Ser Thr Asn Leu Gln Arg
        435                 440                 445

Gly Asn Arg Gln Ala Ala Thr Ala Asp Val Asn Thr Gln Gly Val Leu
    450                 455                 460

Pro Gly Met Val Trp Gln Asp Arg Asp Val Tyr Leu Gln Gly Pro Ile
465                 470                 475                 480

Trp Ala Lys Ile Pro His Thr Asp Gly His Phe His Pro Ser Pro Leu
                485                 490                 495

Met Gly Gly Phe Gly Leu Lys His Pro Pro Pro Gln Ile Leu Ile Lys
            500                 505                 510

Asn Thr Pro Val Pro Ala Asn Pro Ser Thr Thr Phe Ser Ala Ala Lys
        515                 520                 525

Phe Ala Ser Phe Ile Thr Gln Tyr Ser Thr Gly Gln Val Ser Val Glu
    530                 535                 540

Ile Glu Trp Glu Leu Gln Lys Glu Asn Ser Lys Arg Trp Asn Pro Glu
545                 550                 555                 560

Ile Gln Tyr Thr Ser Asn Tyr Asn Lys Ser Val Asn Val Asp Phe Thr
                565                 570                 575

Val Asp Thr Asn Gly Val Tyr Ser Glu Pro Arg Pro Ile Gly Thr Arg
            580                 585                 590

Tyr Leu Thr Arg Asn Leu
        595

<210>  8
<211>  533
<212>  PRT
<213>  adeno-associated virus 2

<400>  8

Met Ala Thr Gly Ser Gly Ala Pro Met Ala Asp Asn Asn Glu Gly Ala
1               5                   10                  15

Asp Gly Val Gly Asn Ser Ser Gly Asn Trp His Cys Asp Ser Thr Trp
            20                  25                  30

Met Gly Asp Arg Val Ile Thr Thr Ser Thr Arg Thr Trp Ala Leu Pro
        35                  40                  45

Thr Tyr Asn Asn His Leu Tyr Lys Gln Ile Ser Ser Gln Ser Gly Ala
    50                  55                  60

Ser Asn Asp Asn His Tyr Phe Gly Tyr Ser Thr Pro Trp Gly Tyr Phe
65                  70                  75                  80

Asp Phe Asn Arg Phe His Cys His Phe Ser Pro Arg Asp Trp Gln Arg
                85                  90                  95

Leu Ile Asn Asn Asn Trp Gly Phe Arg Pro Lys Arg Leu Asn Phe Lys
            100                 105                 110

Leu Phe Asn Ile Gln Val Lys Glu Val Thr Gln Asn Asp Gly Thr Thr
        115                 120                 125

Thr Ile Ala Asn Asn Leu Thr Ser Thr Val Gln Val Phe Thr Asp Ser
    130                 135                 140

Glu Tyr Gln Leu Pro Tyr Val Leu Gly Ser Ala His Gln Gly Cys Leu
145                 150                 155                 160

Pro Pro Phe Pro Ala Asp Val Phe Met Val Pro Gln Tyr Gly Tyr Leu
                165                 170                 175

Thr Leu Asn Asn Gly Ser Gln Ala Val Gly Arg Ser Ser Phe Tyr Cys
            180                 185                 190

Leu Glu Tyr Phe Pro Ser Gln Met Leu Arg Thr Gly Asn Asn Phe Thr
        195                 200                 205

Phe Ser Tyr Thr Phe Glu Asp Val Pro Phe His Ser Ser Tyr Ala His
    210                 215                 220

Ser Gln Ser Leu Asp Arg Leu Met Asn Pro Leu Ile Asp Gln Tyr Leu
225                 230                 235                 240

Tyr Tyr Leu Ser Arg Thr Asn Thr Pro Ser Gly Thr Thr Thr Gln Ser
                245                 250                 255

Arg Leu Gln Phe Ser Gln Ala Gly Ala Ser Asp Ile Arg Asp Gln Ser

            260                 265                 270

Arg Asn Trp Leu Pro Gly Pro Cys Tyr Arg Gln Gln Arg Val Ser Lys
        275                 280                 285

Thr Ser Ala Asp Asn Asn Asn Ser Glu Tyr Ser Trp Thr Gly Ala Thr
    290                 295                 300

Lys Tyr His Leu Asn Gly Arg Asp Ser Leu Val Asn Pro Gly Pro Ala
305                 310                 315                 320

Met Ala Ser His Lys Asp Asp Glu Glu Lys Phe Phe Pro Gln Ser Gly
                325                 330                 335

Val Leu Ile Phe Gly Lys Gln Gly Ser Glu Lys Thr Asn Val Asp Ile
            340                 345                 350

Glu Lys Val Met Ile Thr Asp Glu Glu Glu Ile Arg Thr Thr Asn Pro
        355                 360                 365

Val Ala Thr Glu Gln Tyr Gly Ser Val Ser Thr Asn Leu Gln Arg Gly
    370                 375                 380

Asn Arg Gln Ala Ala Thr Ala Asp Val Asn Thr Gln Gly Val Leu Pro
385                 390                 395                 400

Gly Met Val Trp Gln Asp Arg Asp Val Tyr Leu Gln Gly Pro Ile Trp
                405                 410                 415

Ala Lys Ile Pro His Thr Asp Gly His Phe His Pro Ser Pro Leu Met
            420                 425                 430

Gly Gly Phe Gly Leu Lys His Pro Pro Pro Gln Ile Leu Ile Lys Asn
        435                 440                 445

Thr Pro Val Pro Ala Asn Pro Ser Thr Thr Phe Ser Ala Ala Lys Phe
    450                 455                 460

Ala Ser Phe Ile Thr Gln Tyr Ser Thr Gly Gln Val Ser Val Glu Ile
465                 470                 475                 480

Glu Trp Glu Leu Gln Lys Glu Asn Ser Lys Arg Trp Asn Pro Glu Ile
                485                 490                 495

Gln Tyr Thr Ser Asn Tyr Asn Lys Ser Val Asn Val Asp Phe Thr Val
            500                 505                 510

Asp Thr Asn Gly Val Tyr Ser Glu Pro Arg Pro Ile Gly Thr Arg Tyr
        515                 520                 525

Leu Thr Arg Asn Leu
    530

<210>  9
<211>  744
<212>  PRT
<213>  adeno-associated virus 2

<400>  9

Met Ala Ala Asp Gly Tyr Leu Pro Asp Trp Leu Glu Asp Thr Leu Ser
1               5                   10                  15

Glu Gly Ile Arg Gln Trp Trp Lys Leu Lys Pro Gly Pro Pro Pro Pro
            20                  25                  30

Lys Pro Ala Glu Arg His Lys Asp Asp Ser Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Glu Ala Asp Ala Ala Ala Leu Glu His Asp Lys Ala Tyr Asp
65                  70                  75                  80

Arg Gln Leu Asp Ser Gly Asp Asn Pro Tyr Leu Lys Tyr Asn His Ala
                85                  90                  95

Asp Ala Glu Phe Gln Glu Arg Leu Lys Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Glu Pro Val Lys Thr Ala Pro Gly Lys Lys Arg
    130                 135                 140

Pro Val Glu His Ser Pro Val Glu Pro Asp Ser Ser Ser Gly Thr Gly
145                 150                 155                 160

Lys Ala Gly Gln Gln Pro Ala Arg Lys Arg Leu Asn Phe Gly Gln Thr
                165                 170                 175

Gly Asp Ala Asp Ser Val Pro Asp Pro Gln Pro Leu Gly Gln Pro Pro
            180                 185                 190

Ala Ala Pro Ser Gly Leu Gly Thr Asn Thr Met Ala Thr Gly Ser Gly
        195                 200                 205

Ala Pro Met Ala Asp Asn Asn Glu Gly Ala Asp Gly Val Gly Asn Ser
    210                 215                 220

Ser Gly Asn Trp His Cys Asp Ser Thr Trp Met Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Ser Gln Ser Gly Ala Ser Asn Asp Asn His Tyr
            260                 265                 270

Phe Gly Tyr Ser Thr Pro Trp Gly Tyr Phe Asp Phe Asn Arg Phe His
        275                 280                 285

Cys His Phe Ser Pro Arg Asp Trp Gln Arg Leu Ile Asn Asn Asn Trp
    290                 295                 300

Gly Phe Arg Pro Lys Arg Leu Asn Phe Lys Leu Phe Asn Ile Gln Val
305                 310                 315                 320

Lys Glu Val Thr Gln Asn Asp Gly Thr Thr Thr Ile Ala Asn Asn Leu
                325                 330                 335

Thr Ser Thr Val Gln Val Phe Thr Asp Ser Glu Tyr Gln Leu Pro Tyr
            340                 345                 350

Val Leu Gly Ser Ala His Gln Gly Cys Leu Pro Pro Phe Pro Ala Asp
        355                 360                 365

Val Phe Met Val Pro Gln Tyr Gly Tyr Leu Thr Leu Asn Asn Gly Ser
    370                 375                 380

Gln Ala Val Gly Arg Ser Ser Phe Tyr Cys Leu Glu Tyr Phe Pro Ser
385                 390                 395                 400

Gln Met Leu Arg Thr Gly Asn Asn Phe Thr Phe Ser Tyr Thr Phe Glu
                405                 410                 415

Asp Val Pro Phe His Ser Ser Tyr Ala His Ser Gln Ser Leu Asp Arg
            420                 425                 430

Leu Met Asn Pro Leu Ile Asp Gln Tyr Leu Tyr Tyr Leu Ser Arg Thr
        435                 440                 445

Asn Thr Pro Ser Gly Thr Thr Thr Gln Ser Arg Leu Gln Phe Ser Gln
    450                 455                 460

Ala Gly Ala Ser Asp Ile Arg Asp Gln Ser Arg Asn Trp Leu Pro Gly
465                 470                 475                 480

Pro Cys Tyr Arg Gln Gln Arg Val Ser Lys Thr Ser Ala Asp Asn Asn
                485                 490                 495

Asn Ser Glu Tyr Ser Trp Thr Gly Ala Thr Lys Tyr His Leu Asn Gly
            500                 505                 510

Arg Asp Ser Leu Val Asn Pro Gly Pro Ala Met Ala Ser His Lys Asp
        515                 520                 525

Asp Glu Glu Lys Phe Phe Pro Gln Ser Gly Val Leu Ile Phe Gly Lys
    530                 535                 540

Gln Gly Ser Glu Lys Thr Asn Val Asp Ile Glu Lys Val Met Ile Thr
545                 550                 555                 560

Asp Glu Glu Glu Ile Arg Thr Thr Asn Pro Val Ala Thr Glu Gln Tyr
                565                 570                 575

Gly Ser Val Ser Thr Asn Leu Gln Arg Gly Gln Arg Gly Glu Ser Gly
            580                 585                 590

His Gly Tyr Phe Ala Gln Ala Ala Thr Ala Asp Val Asn Thr Gln Gly
        595                 600                 605

Val Leu Pro Gly Met Val Trp Gln Asp Arg Asp Val Tyr Leu Gln Gly
    610                 615                 620

Pro Ile Trp Ala Lys Ile Pro His Thr Asp Gly His Phe His Pro Ser
625                 630                 635                 640

Pro Leu Met Gly Gly Phe Gly Leu Lys His Pro Pro Pro Gln Ile Leu
                645                 650                 655

Ile Lys Asn Thr Pro Val Pro Ala Asn Pro Ser Thr Thr Phe Ser Ala
            660                 665                 670

Ala Lys Phe Ala Ser Phe Ile Thr Gln Tyr Ser Thr Gly Gln Val Ser
        675                 680                 685

Val Glu Ile Glu Trp Glu Leu Gln Lys Glu Asn Ser Lys Arg Trp Asn
    690                 695                 700

Pro Glu Ile Gln Tyr Thr Ser Asn Tyr Asn Lys Ser Val Asn Val Asp
705                 710                 715                 720

Phe Thr Val Asp Thr Asn Gly Val Tyr Ser Glu Pro Arg Pro Ile Gly
                725                 730                 735

Thr Arg Tyr Leu Thr Arg Asn Leu
            740

<210>  10
<211>  8359
<212>  DNA
<213>  adeno-associated virus 2

<400>  10
catcatcaat aatatacctt attttggatt gaagccaata tgataatgag ggggtggagt      60

ttgtgacgtg gcgcggggcg tgggaacggg gcgggtgacg tagtagctct agaggtcctg     120

tattagaggt cacgtgagtg ttttgcgaca ttttgcgaca ccatgtggtc acgctgggta     180

tttaagcccg agtgagcacg cagggtctcc attttgaagc gggaggtttg aacgcgcagc     240

cgccatgccg gggttttacg agattgtgat taaggtcccc agcgaccttg acgagcatct     300

gcccggcatt tctgacagct ttgtgaactg ggtggccgag aaggaatggg agttgccgcc     360

agattctgac atggatctga atctgattga gcaggcaccc ctgaccgtgg ccgagaagct     420

gcagcgcgac tttctgacgg aatggcgccg tgtgagtaag gcaccggagg cccttttctt     480

tgtgcaattt gagaagggag agagctactt ccacatgcac gtgctcgtgg aaaccaccgg     540

ggtgaaatcc atggttttgg gacgtttcct gagtcagatt cgcgaaaaac tgattcagag     600

aatttaccgc gggatcgagc cgactttgcc aaactggttc gcggtcacaa agaccagaaa     660

tggcgccgga ggcgggaaca aggtggtgga tgagtgctac atccccaatt acttgctccc     720

caaaacccag cctgagctcc agtgggcgtg gactaatatg gaacagtatt taagcgcctg     780

tttgaatctc acggagcgta aacggttggt ggcgcagcat ctgacgcacg tgtcgcagac     840

gcaggagcag aacaaagaga atcagaatcc caattctgat gcgccggtga tcagatcaaa     900

aacttcagcc aggtacatgg agctggtcgg gtggctcgtg gacaagggga ttacctcgga     960

gaagcagtgg atccaggagg accaggcctc atacatctcc ttcaatgcgg cctccaactc    1020

gcggtcccaa atcaaggctg ccttggacaa tgcgggaaag attatgagcc tgactaaaac    1080

cgcccccgac tacctggtgg gccagcagcc cgtggaggac atttccagca atcggattta    1140

taaaattttg gaactaaacg ggtacgatcc ccaatatgcg gcttccgtct ttctgggatg    1200

ggccacgaaa aagttcggca agaggaacac catctggctg tttgggcctg caactaccgg    1260

gaagaccaac atcgcggagg ccatagccca cactgtgccc ttctacgggt gcgtaaactg    1320

gaccaatgag aactttccct tcaacgactg tgtcgacaag atggtgatct ggtgggagga 1380

ggggaagatg accgccaagg tcgtggagtc ggccaaagcc attctcggag gaagcaaggt 1440

gcgcgtggac cagaaatgca agtcctcggc ccagatagac ccgactcccg tgatcgtcac 1500

ctccaacacc aacatgtgcg ccgtgattga cgggaactca acgaccttcg aacaccagca 1560

gccgttgcaa gaccggatgt tcaaatttga actcacccgc cgtctggatc atgactttgg 1620

gaaggtcacc aagcaggaag tcaaagactt tttccggtgg gcaaaggatc acgtggttga 1680

ggtggagcat gaattctacg tcaaaaaggg tggagccaag aaaagacccg cccccagtga 1740

cgcagatata agtgagccca aacgggtgcg cgagtcagtt gcgcagccat cgacgtcaga 1800

cgcggaagct tcgatcaact acgcagacag gtaccaaaac aaatgttctc gtcacgtggg 1860

catgaatctg atgctgtttc cctgcagaca atgcgagaga atgaatcaga attcaaatat 1920

ctgcttcact cacggacaga aagactgttt agagtgcttt cccgtgtcag aatctcaacc 1980

cgtttctgtc gtcaaaaagg cgtatcagaa actgtgctac attcatcata tcatgggaaa 2040

ggtgccagac gcttgcactg cctgcgatct ggtcaatgtg gatttggatg actgcatctt 2100

tgaacaataa atgatttaaa tcaggtatgg ctgccgatgg ttatcttcca gattggctcg 2160

aggacactct ctctgaagga ataagacagt ggtggaagct caaacctggc ccaccaccac 2220

caaagcccgc agagcggcat aaggacgaca gcaggggtct tgtgcttcct gggtacaagt 2280

acctcggacc cttcaacgga ctcgacaagg gagagccggt caacgaggca gacgccgcgg 2340

ccctcgagca cgacaaagcc tacgaccggc agctcgacag cggagacaac ccgtacctca 2400

agtacaacca cgccgacgcg gagtttcagg agcgccttaa agaagatacg tcttttgggg 2460

gcaacctcgg acgagcagtc ttccaggcga aaaagagggt tcttgaacct ctgggcctgg 2520

ttgaggaacc tgttaagacg gctccgggaa aaaagaggcc ggtagagcac tctcctgtgg 2580

agccagactc ctcctcggga accggaaagg cgggccagca gcctgcaaga aaaagattga 2640

attttggtca gactggagac gcagactcag tacctgaccc ccagcctctc ggacagccac 2700

cagcagcccc ctctggtctg ggaactaata cgatggctac aggcagtggc gcaccaatgg 2760

cagacaataa cgagggcgcc gacggagtgg gtaattcctc gggaaattgg cattgcgatt 2820

ccacatggat gggcgacaga gtcatcacca ccagcacccg aacctgggcc ctgcccacct 2880

acaacaacca cctctacaaa caaatttcca gccaatcagg agcctcgaac gacaatcact 2940

actttggcta cagcacccct tgggggtatt ttgacttcaa cagattccac tgccactttt 3000

caccacgtga ctggcaaaga ctcatcaaca acaactgggg attccgaccc aagagactca 3060

acttcaagct ctttaacatt caagtcaaag aggtcacgca gaatgacggt acgacgacga 3120

ttgccaataa ccttaccagc acggttcagg tgtttactga ctcggagtac cagctcccgt 3180

acgtcctcgg ctcggcgcat caaggatgcc tcccgccgtt cccagcagac gtcttcatgg 3240

tgccacagta tggatacctc accctgaaca acgggagtca ggcagtagga cgctcttcat 3300

tttactgcct ggagtacttt ccttctcaga tgctgcgtac cggaaacaac tttaccttca 3360

gctacacttt tgaggacgtt cctttccaca gcagctacgc tcacagccag agtctggacc 3420

gtctcatgaa tcctctcatc gaccagtacc tgtattactt gagcagaaca aacactccaa 3480

gtggaaccac cacgcagtca aggcttcagt tttctcaggc cggagcgagt gacattcggg 3540

accagtctag gaactggctt cctggaccct gttaccgcca gcagcgagta tcaaagacat 3600

ctgcggataa caacaacagt gaatactcgt ggactggagc taccaagtac cacctcaatg 3660

gcagagactc tctggtgaat ccgggcccgg ccatggcaag ccacaaggac gatgaagaaa 3720

agttttttcc tcagagcggg gttctcatct ttgggaagca aggctcagag aaaacaaatg 3780

tggacattga aaaggtcatg attacagacg aagaggaaat caggacaacc aatcccgtgg 3840

ctacggagca gtatggttct gtatctacca acctccagag aggccagaga ggcgagtctg 3900

gtcatggtta ttttgcccag gcggccaccg cagatgtcaa cacacaaggc gttcttccag 3960

gcatggtctg gcaggacaga gatgtgtacc ttcaggggcc catctgggca aagattccac 4020

acacggacgg acattttcac ccctctcccc tcatgggtgg attcggactt aaacaccctc 4080

ctccacagat tctcatcaag aacaccccgg tacctgcgaa tccttcgacc accttcagtg 4140

cggcaaagtt tgcttccttc atcacacagt actccacggg acaggtcagc gtggagatcg 4200

agtgggagct gcagaaggaa aacagcaaac gctggaatcc cgaaattcag tacacttcca 4260

actacaacaa gtctgttaat gtggacttta ctgtggacac taatggcgtg tattcagagc 4320

ctcgccccat tggcaccaga tacctgactc gtaatctgta attgcttgtt aatcaataaa 4380

ccgtttaatt cgtttcagtt gaactttggt ctctgcgtat ttctttctta tctagtttcc 4440

atgctctaga ggtcctgtat tagaggtcac gtgagtgttt tgcgacattt tgcgacacca 4500

tgtggtcacg ctgggtattt aagcccgagt gagcacgcag ggtctccatt ttgaagcggg 4560

aggtttgaac gcgcagccac cacggcgggg ttttacgaga ttgtgattaa ggtccccagc 4620

gaccttgacg agcatctgcc cggcatttct gacagctttg tgaactgggt ggccgagaag 4680

gaatgggagt tgccgccaga ttctgacatg gatctgaatc tgattgagca ggcacccctg 4740

accgtggccg agaagctgca tcgctggcgt aatagcgaag aggcccgcac cgatcgccct 4800

tcccaacagt tgcgcagcct gaatggcgaa tggcgattcc gttgcaatgg ctggcggtaa 4860

tattgttctg gatattacca gcaaggccga tagtttgagt tcttctactc aggcaagtga 4920

tgttattact aatcaaagaa gtattgcgac aacggttaat ttgcgtgatg gacagactct 4980

tttactcggt ggcctcactg attataaaaa cacttctcag gattctggcg taccgttcct 5040

gtctaaaatc cctttaatcg gcctcctgtt tagctcccgc tctgattcta acgaggaaag 5100

cacgttatac gtgctcgtca aagcaaccat agtacgcgcc ctgtagcggc gcattaagcg 5160

cggcgggtgt ggtggttacg cgcagcgtga ccgctacact tgccagcgcc ctagcgcccg 5220

ctcctttcgc tttcttccct tcctttctcg ccacgttcgc cggctttccc cgtcaagctc 5280

taaatcgggg gctcccttta gggttccgat ttagtgcttt acggcacctc gaccccaaaa 5340

aacttgatta gggtgatggt tcacgtagtg ggccatcgcc ctgatagacg gtttttcgcc 5400

ctttgacgtt ggagtccacg ttctttaata gtggactctt gttccaaact ggaacaacac 5460

tcaaccctat ctcggtctat tcttttgatt tataagggat tttgccgatt tcggcctatt 5520

ggttaaaaaa tgagctgatt taacaaaaat ttaacgcgaa ttttaacaaa atattaacgt 5580

ttacaattta aatatttgct tatacaatct tcctgttttt ggggcttttc tgattatcaa 5640

ccggggtaca tatgattgac atgctagttt tacgattacc gttcatcgat tctcttgttt 5700

gctccagact ctcaggcaat gacctgatag cctttgtaga gacctctcaa aaatagctac 5760

cctctccggc atgaatttat cagctagaac ggttgaatat catattgatg gtgatttgac 5820

tgtctccggc ctttctcacc cgtttgaatc tttacctaca cattactcag gcattgcatt 5880

taaaatatat gagggttcta aaaattttta tccttgcgtt gaaataaagg cttctcccgc 5940

aaaagtatta cagggtcata atgtttttgg tacaaccgat ttagctttat gctctgaggc 6000

tttattgctt aattttgcta attctttgcc ttgcctgtat gatttattgg atgttggaat 6060

tcctgatgcg gtattttctc cttacgcatc tgtgcggtat ttcacaccgc atatggtgca 6120

ctctcagtac aatctgctct gatgccgcat agttaagcca gccccgacac ccgccaacac 6180

ccgctgacgc gccctgacgg gcttgtctgc tcccggcatc cgcttacaga caagctgtga 6240

ccgtctccgg gagctgcatg tgtcagaggt tttcaccgtc atcaccgaaa cgcgcgagac 6300

gaaagggcct cgtgatacgc ctatttttat aggttaatgt catgataata atggtttctt 6360

agacgtcagg tggcactttt cggggaaatg tgcgcggaac ccctatttgt ttatttttct 6420

aaatacattc aaatatgtat ccgctcatga gacaataacc ctgataaatg cttcaataat 6480

attgaaaaag gaagagtatg agtattcaac atttccgtgt cgcccttatt cccttttttg 6540

cggcattttg ccttcctgtt tttgctcacc cagaaacgct ggtgaaagta aaagatgctg 6600

aagatcagtt gggtgcacga gtgggttaca tcgaactgga tctcaacagc ggtaagatcc 6660

ttgagagttt tcgccccgaa gaacgttttc caatgatgag cacttttaaa gttctgctat 6720

gtggcgcggt attatcccgt attgacgccg ggcaagagca actcggtcgc cgcatacact 6780

attctcagaa tgacttggtt gagtactcac cagtcacaga aaagcatctt acggatggca 6840

tgacagtaag agaattatgc agtgctgcca taaccatgag tgataacact gcggccaact 6900

tacttctgac aacgatcgga ggaccgaagg agctaaccgc ttttttgcac aacatggggg 6960

atcatgtaac tcgccttgat cgttgggaac cggagctgaa tgaagccata ccaaacgacg 7020

agcgtgacac cacgatgcct gtagcaatgg caacaacgtt gcgcaaacta ttaactggcg 7080

aactacttac tctagcttcc cggcaacaat taatagactg gatggaggcg gataaagttg 7140

caggaccact tctgcgctcg gcccttccgg ctggctggtt tattgctgat aaatctggag 7200

ccggtgagcg tgggtctcgc ggtatcattg cagcactggg gccagatggt aagccctccc 7260

gtatcgtagt tatctacacg acggggagtc aggcaactat ggatgaacga aatagacaga 7320

tcgctgagat aggtgcctca ctgattaagc attggtaact gtcagaccaa gtttactcat 7380

atatacttta gattgattta aaacttcatt tttaatttaa aaggatctag gtgaagatcc 7440

tttttgataa tctcatgacc aaaatccctt aacgtgagtt ttcgttccac tgagcgtcag 7500

accccgtaga aaagatcaaa ggatcttctt gagatccttt ttttctgcgc gtaatctgct 7560

gcttgcaaac aaaaaaacca ccgctaccag cggtggtttg tttgccggat caagagctac 7620

caactctttt tccgaaggta actggcttca gcagagcgca gataccaaat actgtccttc 7680

tagtgtagcc gtagttaggc caccacttca agaactctgt agcaccgcct acatacctcg 7740

ctctgctaat cctgttacca gtggctgctg ccagtggcga taagtcgtgt cttaccgggt 7800

tggactcaag acgatagtta ccggataagg cgcagcggtc gggctgaacg gggggttcgt 7860

gcacacagcc cagcttggag cgaacgacct acaccgaact gagataccta cagcgtgagc 7920

tatgagaaag cgccacgctt cccgaaggga gaaaggcgga caggtatccg gtaagcggca 7980

gggtcggaac aggagagcgc acgagggagc ttccaggggg aaacgcctgg tatctttata 8040

gtcctgtcgg gtttcgccac ctctgacttg agcgtcgatt tttgtgatgc tcgtcagggg 8100

ggcggagcct atggaaaaac gccagcaacg cggccttttt acggttcctg gccttttgct 8160

ggccttttgc tcacatgttc tttcctgcgt tatcccctga ttctgtggat aaccgtatta 8220

ccgcctttga gtgagctgat accgctcgcc gcagccgaac gaccgagcgc agcgagtcag 8280

tgagcgagga agcggaagag cgcccaatac gcaaaccgcc tctccccgcg cgttggccga 8340

ttcattaatg cagaattcc 8359

<210> 11
<211> 56
<212> DNA
<213> Artificial Sequence

<220>
<223> PCR Primer


<220>
<221> misc_feature
<222> (18)..(38)
<223> n is a, c, g, or t

<400>  11
cagtcggcca gagaggcnnn nnnnnnnnnn nnnnnnnngc ccaggcggct gacgag          56


<210>  12
<211>  16
<212>  DNA
<213>  Artificial Sequence

<220>
<223>  PCR Primer

<400>  12
ctcgtcagcc gcctgg                                                      16


<210>  13
<211>  26
<212>  DNA
<213>  Artificial Sequence

<220>
<223>  PCR Primer

<400>  13
gcagtatggt tctgtatcta ccaacc                                           26


<210>  14
<211>  22
<212>  DNA
<213>  Artificial Sequence

<220>
<223>  PCR Primer

<400>  14
gcctggaaga acgccttgtg tg                                               22


<210>  15
<211>  22
<212>  DNA
<213>  Artificial Sequence

<220>
<223>  PCR Primer

<400>  15
atggcaagcc acaaggacga tg                                               22


<210>  16
<211>  22
<212>  DNA
<213>  Artificial Sequence

<220>
<223>  PCR Primer

<400>  16
cgtggagtac tgtgtgatga ag                                               22

<210>  17
<211>  23
<212>  DNA
<213>  Artificial Sequence

<220>
<223>  PCR Primer

<400>  17
ggttctcatc tttgggaagc aag                                               23


<210>  18
<211>  21
<212>  DNA
<213>  Artificial Sequence

<220>
<223>  PCR Primer

<400>  18
tgatgagaat ctgtggagga g                                                 21


<210>  19
<211>  19
<212>  DNA
<213>  Artificial Sequence

<220>
<223>  PCR Primer

<400>  19
ggcggagttg ttacgacat                                                    19


<210>  20
<211>  20
<212>  DNA
<213>  Artificial Sequence

<220>
<223>  PCR Primer

<400>  20
gggactttcc ctacttggca                                                   20